# EUROPEAN PATENT APPLICATION

(11) **EP 3 736 286 A1**
(43) Date of publication of application: **11.11.2020**
(21) Application number: 19173440.9
(22) Date of filing: 09.05.2019
(51) Int. Cl.: C07K 14/755, A61K 38/37, C12N 15/00

(54) **SINGLE CHAIN FACTOR VIII MOLECULE**

(71) Applicant: Biotest AG, 63303 Dreieich (DE)
(72) Inventor: KISTNER, Steffen, 60599 Frankfurt/Main (DE); UNGERER, Christopher, 63225 Langen (DE); DAUFENBACH, Jens, 55120 Mainz (DE); HERBENER, Peter, 35274 Kirchhain (DE)
(74) Representative: Moré, Solveig Helga

(57) **Abstract**

The present invention relates to a recombinant Factor VIII protein comprising, in a single chain, a heavy chain portion comprising an A1 and an A2 domain and a light chain portion comprising an A3, C1 and C2 domain of Factor VIII, wherein the B-domain is partially deleted in two deletions, the first leading to the presence of a defined processing sequence cleavable by thrombin, and the second leading to absence of the furin cleavage recognition site at position R1664-R1667. An internal fragment of the B-domain is maintained. Nucleic acids encoding said protein, host cells and methods of preparing the protein are also provided, as well as a pharmaceutical composition comprising the protein, nucleic acid or host cell, which may be used for treatment of hemophilia A.

## Description

The present invention relates to a recombinant Factor VIII (FVIII) protein comprising, in a single chain, a heavy chain portion comprising an A1 and an A2 domain and a light chain portion comprising an A3, C1 and C2 domain of Factor VIII, wherein the B-domain is partially deleted in two deletions, the first leading to the presence of a defined processing sequence cleavable by thrombin, and the second leading to absence of the furin cleavage recognition site. An internal fragment of the B-domain is maintained. Nucleic acids encoding said protein, host cells and methods of preparing the protein are also provided, as well as a pharmaceutical composition comprising the protein, nucleic acid or host cell, which may be used for treatment of hemophilia A.

FVIII is an important co-factor in the coagulation cascade. Natural FVIII is synthesized as a single chain protein and is afterwards cleaved by intracellular proteases. The secreted FVIII is a heterodimer with a truncated B domain. If the coagulation cascade is activated, FVIII is cleaved by thrombin at three positions, leading to a heterotrimer and loss of the B domain (heterotrimeric FVIIIa). The heterotrimer builds a complex with the activated coagulation Factor IXa and coagulation Factor X, thereby promoting the activation of the latter one.

The human natural FVIII single chain protein consists of 2351 amino acids. The first 19 amino acids comprise the signal sequence which is cleaved prior to secretion, leading to a FVIII molecule comprising 2332 amino acids. Additionally, cleavage occurs at the C-terminal part of the B domain, leading to a mature heterodimeric FVIII. The heavy chain of the heterodimer comprises the domains A1 (residues 20-355), A2 (392-729) and B (760-1667), whereas the light chain comprises the domains A3 (1709-2038), C1 (2039-2191) and C2 (2192-2351). Moreover, there are three spacer regions: a1 (356 - 391), a2 (730-759) und a3 (1668 - 1708).

Annotation of amino acids in the FVIII molecule differs between authors. This is due to the 19 amino acid signal sequence which can be included into the amino acid count or can be omitted (the above annotation is based on inclusion of the signal sequence). This variation of plus or minus 19 amino acids is a frequent difference in numeration for full-length FVIII sequences in the prior literature. For B-domain deleted FVIII constructs, the deletion may also lead to a shift in numeration. For the heavy chain, the numeration correlates with the numeration of the full-length FVIII. From the B-domain deletion on the numeration of the light chain is either kept the same as for the full-length FVIII molecule (e.g. Q763 in front of the deletion is followed by D1582 after the deletion) or can be continued as if no deletion has occurred (e.g., Q763 is followed by D764 despite missing amino acids). The continued numeration complicates the comparison of amino acid sequences, if it is not known how many amino acids were deleted. In the present specification, the numeration of the full-length FVIII molecule is maintained despite (partial) B-domain deletion.

Hemophilia A is a genetic bleeding disorder with deficiency in clotting factor VIII linked to the X-chromosome, occurring in 1 of 5000 newborn males. However, Hemophilia A can also occur spontaneously due to an auto-immune response against FVIII. Patients with Hemophilia A suffer from longer bleeding durations, spontaneous and internal bleedings, affecting their everyday life.

Hemophilia A patients are generally treated by administration of FVIII. Depending on the severity of the disease (mild, moderate or severe) treatment is on demand or prophylactic. Therapeutic FVIII products are either purified from human plasma (pFVIII) or the products are produced recombinantly in cell culture (rFVIII).

During the development of recombinant FVIII molecules for therapy, B-domain deleted FVIII molecules have been designed, because the B-domain is not crucial for the functionality of FVIII in clotting. This predominantly leads to a reduction in size, which facilitates production and storage. The most common B-domain deleted FVIII product is ReFacto® or ReFacto AF® produced by Pfizer. This FVIII variant lacks 894 amino acids of the B domain. However, the furin cleavage recognition site at the end of the B-domain is still present and ReFacto AF® thus is a double chain protein. The review article of Kenneth Lieuw (J Blood Med. 2017; 8: 67-73) highlights some of the differences between Factor VIII products currently available.

Currently, most rFVIII therapeutics are based on the classical double chain FVIII structure comprising the heavy chain (domains A1-a1-A2-a2-[B]) and the light chain (domains a3-A3-C1-C2). During expression, both heavy and light chains are initially translated from one mRNA molecule, but the amino acid sequence comprises a furin cleavage recognition site (R1664-R1667) at the end of the B-domain enabling intracellular proteolytic processing by cleavage after R1667 and separation of both domains. However, heavy and light chains are typically non-covalently connected via a divalent cation.

More recently, recombinant single chain FVIII proteins have been developed. Such B-domain deleted FVIII proteins are designed as a B-domain truncated recombinant FVIII molecule, wherein the light and heavy chains are covalently linked in order to form a stable single chain FVIII molecule (Schmidbauer S. et al., Thromb Res 2015; 136: 388-395). Because according to this approach, the heavy and light chain segments are linked via a strong covalent bond, the segments are less likely to dissociate (Pabinger-Fasching, I., Thromb Res 2016; 141S3: 2-4). The single chain molecules can be isolated as a pure and homogenous compound. It has been observed that the half-life of single chain FVIII molecules was approximately twice that of full-length recombinant FVIII molecules (Zollner S. et al., Thromb Res 2014; 134: 125-131). However, some clinical results with a recombinant, single chain Factor VIII molecule (AFSTYLA®) showed only a marginally enhanced half-life of the single chain molecule compared to a double chain molecule. Nguyen et al. (J Thromb Haemost. 2017 Jan; 15(1): 110-121) describe novel factor VIII variants with a modified furin cleavage recognition site (furin cleavage recognition site pos. 1664-1667). Variants with mutations at the furin cleavage recognition site are primarily secreted in a single polypeptide chain form and show improved expression compared to double chain B-domain deleted variants.

WO 2017/123961 A1 discloses Factor VIII variants with a B-domain deletion, wherein one or two amino acids at positions 1676 or 1677 are substituted, modified or deleted compared to wild type FVIII. Optionally, the variants may further include a mutated PACE-furin cleavage recognition site (HHQR or RHQR at pos. 1664 - 1667).

US 6,316,226 B1 discloses a polypeptide having FVIII activity, wherein the polypeptide has an internal deletion of amino acids 760 through 1687 as compared to human FVIII.

WO 2014/008480 A2 discloses single chain FVIII molecules with full or partial deletion of the B domain. A preferred molecule has a deletion of pos. 784 - 1677 and substitutions R1683A and R1686A (SEQ ID No. 8 in WO 2014/008480 A2).

WO 2013/057219 A1 describes a FVIII molecule wherein a first amino acid selected from the amino acids at positions 760 to 1666 is fused with a second amino acid selected from the amino acids at positions 1668 to 1709. The proteolytic cleavage site between Arg1667 and Glu1668 and, if present, the proteolytic cleavage site between Arg1332 and Ala1333 is inactivated.

WO 2004/067566 A1 discloses FVIII polypeptides comprising an internal deletion of one or more amino acids between 1668 and 1707 fused to any amino acid sequence in B domain from about 760 to 801.

US 6,316,226 B1 discloses a DNA encoding a Factor VIII analog, wherein the analog has an internal deletion of amino acids 760 through 1687 as compared to human Factor VIII.

Further single chain FVIII molecules are described e.g. in WO 88/09813 A1 and WO 2011/041770 A1.

WO 2014/210547 A1, WO 2014/026954 A1, WO 2013/122617 A1, WO 2013/106787 A1, WO 2015/106052 A1 disclose further single chain FVIII variants with modifications or deletions of the furin cleavage recognition site. In summary, single chain variants of FVIII have certain advantages regarding their purification and production, in particular when intended for pharmaceutical use. Purification of wild type FVIII is challenging due to the noncovalent bond between the FVIII heavy and light chains. Furthermore, corresponding noncovalent bonds may also be formed with FVIII fragments. As a result, purification can result in co-purification of fragments and non-stochiometric purification of heavy and light chain, all of which is undesirable for a pharmaceutical product, which ought to be pure, well-defined, and manufactured by a robust process.

However, present single chain FVIII variants suffer from certain disadvantages, such as low expression levels and impaired biological activity. Therefore, there is a need for further and improved FVIII single chain variants with high expression levels and improved biological activity.

In light of the prior art, the inventors addressed the problem of developing improved single chain Factor VIII molecules with an improved and balanced profile of properties. E.g. the molecules allow for easy purification, such as by size exclusion chromatography, and exhibit a high level of expression, while being stable and showing well-retained biological function, including an improved high specific activity.

### Summary of the Invention

In a first embodiment, the invention provides a recombinant Factor VIII protein comprising, in a single chain, a heavy chain portion comprising an A1 and an A2 domain and a light chain portion comprising an A3, C1 and C2 domain of Factor VIII, wherein,
a) in said recombinant Factor VIII protein, 894 amino acids corresponding to consecutive amino acids between F761 and P1659 of wild type Factor VIII as defined in SEQ ID NO: 1 are deleted, leading to a first deletion;
b) said recombinant Factor VIII protein comprises, spanning the site of the first deletion, a processing sequence comprising SEQ ID NO: 2 or a sequence having at most one amino acid substitution in SEQ ID NO: 2, wherein said processing sequence comprises a first thrombin cleavage site;
c) in said recombinant Factor VIII protein, at least the amino acids corresponding to amino acids R1664 to R1667 of wild type Factor VIII are deleted, leading to a second deletion; and
d) said recombinant Factor VIII protein comprises, C-terminal to the second deletion and N-terminal of the A3 domain, a second thrombin cleavage site.

In a second embodiment, the invention provides a recombinant Factor VIII protein comprising, in a single chain, a heavy chain portion comprising an A1 and an A2 domain and a light chain portion comprising an A3, C1 and C2 domain of Factor VIII, wherein,
a) said recombinant Factor VIII protein comprises a processing sequence comprising SEQ ID NO: 2 or a sequence having at most one amino acid substitution in SEQ ID NO: 2, wherein said processing sequence comprises a first thrombin cleavage site;
b) optionally, directly C-terminal to said processing sequence, said Factor VIII protein comprises a heterologous sequence;
c) directly C-terminal to said processing sequence, or, if present, directly C-terminal to said heterologous sequence, said Factor VIII protein comprises a merging sequence having at least 90% sequence identity to a sequence selected from the group consisting of SEQ ID NO: 9, SEQ ID NO: 10 and SEQ ID NO: 11; and
d) said recombinant Factor VIII protein comprises, C-terminal to SEQ ID NO: 9-11, a second thrombin cleavage site.

In a third embodiment, the Factor VIII protein of the second embodiment (embodiment 2) also is a protein of the first embodiment (embodiment 1).

In a fourth embodiment, the recombinant Factor VIII protein of any of embodiments 1-3, comprises a sequence of SEQ ID NO: 9.

In a fifth embodiment, in the recombinant Factor VIII protein of any of embodiments 1-4, amino acids corresponding to
i) F761 and S1656;
ii) S762 and Q1657;
iii) Q763 and N1658; or
iv) N764 and P1659
of wild type Factor VIII as defined in SEQ ID NO: 1 are adjacent to each other, wherein said amino acids are part of the sequence defined in SEQ ID NO: 2. This is due to the first deletion as defined in the first embodiment.

In a sixth embodiment, in the recombinant Factor VIII protein of any one of embodiments 1-5, the processing sequence is SEQ ID NO: 2 or a sequence having at most one amino acid substitution in said sequence, wherein, optionally, the F, the S C-terminal to the F, the Q or the N are substituted.

In a seventh embodiment, in the recombinant Factor VIII protein of any one of embodiments 1-5, the processing sequence is SEQ ID NO: 3 or a sequence having at most one amino acid substitution in said sequence, wherein, optionally, the F, the S C-terminal to the F, the Q or the N are substituted.

In an eighth embodiment, in the recombinant Factor VIII protein of any one of embodiments 1-5, the processing sequence is SEQ ID NO: 4 or a sequence having at most one amino acid substitution in said sequence, wherein, optionally, the F, the S C-terminal to the F, the Q or the N are substituted.

In a ninth embodiment, in the recombinant Factor VIII protein of any one of embodiments 1-5 or 8, the processing sequence is selected from the group consisting of SEQ ID NO: 4, 5, 6, 7 or 8.

In a tenth embodiment, in the recombinant Factor VIII protein of embodiment 9, the processing sequence is SEQ ID NO: 5. In an eleventh embodiment, in the recombinant Factor VIII protein of embodiment 9, the processing sequence is SEQ ID NO: 6. In a twelfth embodiment, in the recombinant Factor VIII protein of embodiment 9, the processing sequence is SEQ ID NO: 7. In a thirteenth embodiment, in the recombinant Factor VIII protein of embodiment 9, the processing sequence is SEQ ID NO: 8. In a fourteenth embodiment, in the recombinant Factor VIII protein of embodiment 9, the processing sequence is SEQ ID NO: 4.

In a fifteenth embodiment, in the recombinant Factor VIII protein of any one of the preceding embodiments, said processing sequence is directly N-terminal to an amino acid corresponding to Q1675, I1681 or E1690 of wild type Factor VIII.

In a sixteenth embodiment, in the recombinant Factor VIII protein of any one of the preceding embodiments, the amino acids corresponding to amino acids K1663 to L1674 of wild type Factor VIII are deleted, leading to a second deletion.

In a seventeenth embodiment, in the recombinant Factor VIII protein of any one of embodiments 1-16, due to the second deletion, amino acids corresponding to amino acids V1661 and L1674 of wild type Factor VIII as defined in SEQ ID NO: 1 are adjacent to each other, or amino acids corresponding to amino acids L1662 and Q1675 of wild type Factor VIII are adjacent to each other. Both options may result in the same sequence.

In an eighteenth embodiment, in the recombinant Factor VIII protein of any one of embodiments 1-16, due to the second deletion, amino acids corresponding to amino acids V1661 and 11681 of wild type Factor VIII as defined in SEQ ID NO: 1 are adjacent to each other.

In a nineteenth embodiment, in the recombinant Factor VIII protein of any one of embodiments 1-16, due to the second deletion, amino acids corresponding to amino acids P1660 and 11681 of wild type Factor VIII as defined in SEQ ID NO: 1 are adjacent to each other.

In a twentieth embodiment, in the recombinant Factor VIII protein of any one of embodiments 1-16, due to the second deletion, amino acids corresponding to amino acids V1661 and E1690 of wild type Factor VIII as defined in SEQ ID NO: 1 are adjacent to each other.

In a 21^{st} embodiment, the recombinant Factor VIII protein of any one of the preceding embodiments does not comprise a furin cleavage recognition site.

In a 22^{nd} embodiment, the recombinant Factor VIII protein of any one of the preceding embodiments does not comprise a sequence having more than 75%, preferably, more than 50% sequence identity to the furin cleavage recognition site RHQR between the processing sequence and the merging sequence. The sequence corresponding to SEQ ID NO: 15, which comprises said furin cleavage recognition site, may be deleted. Optionally, the recombinant Factor VIII protein of any one of the preceding embodiments does not comprise a sequence having more than 30% sequence identity to SEQ ID NO: 15. Alternatively, it does not comprise a sequence having more than 40% sequence identity to SEQ ID NO: 15.

In a 23^{rd} embodiment, the recombinant Factor VIII protein of any one of the preceding embodiments comprises a processing sequence and, C-terminal to said processing sequence, a merging sequence, wherein the processing sequence is selected from the group comprising SEQ ID NO: 2, 3, 4, 5, 6, 7 or 8, and the merging sequence is selected from the group comprising SEQ ID NO: 12, 13 or 14.

In a 24^{th} embodiment, in the recombinant Factor VIII protein of the 23^{rd} embodiment, the processing sequence is SEQ ID NO: 4 and the merging sequence SEQ ID NO: 12. In a 25^{th} embodiment, in the recombinant Factor VIII protein of the 23^{rd} embodiment, the processing sequence is SEQ ID NO: 5 and the merging sequence SEQ ID NO: 12. In a 26^{th} embodiment, in the recombinant Factor VIII protein of the 23^{rd} embodiment, the processing sequence is SEQ ID NO: 6 and the merging sequence of SEQ ID NO: 12. In a 27^{th} embodiment, in the recombinant Factor VIII protein of the 23^{rd} embodiment, the processing sequence is SEQ ID NO: 7 and the merging sequence of SEQ ID NO: 12. In a 28^{th} embodiment, in the recombinant Factor VIII protein of the 23^{rd} embodiment, the processing sequence is SEQ ID NO: 8 and the merging sequence SEQ ID NO: 12. In a 29^{th} embodiment, in the recombinant Factor VIII protein of the 23^{rd} embodiment, the processing sequence is SEQ ID NO: 3 and the merging sequence of SEQ ID NO: 13. In a 30^{th} embodiment, in the recombinant Factor VIII protein of the 23^{rd} embodiment, the processing sequence is SEQ ID NO: 2 and the merging sequence SEQ ID NO: 13. In a 31^{st} embodiment, in the recombinant Factor VIII protein of the 23^{rd} embodiment, the processing sequence is SEQ ID NO: 3 and the merging sequence SEQ ID NO: 14. In a 32^{nd} embodiment, in the recombinant Factor VIII protein of any one of embodiments 23-31, the merging sequence is directly C-terminal to said processing sequence.

In a 33^{rd} embodiment, the recombinant Factor VIII protein of any one of the preceding embodiments is a fusion protein with at least one heterologous fusion partner selected from the group consisting of an Fc region, an albumin-binding sequence, albumin, PAS polypeptides, HAP polypeptides, the C-terminal peptide of the beta subunit of chorionic gonadotropin, albumin-binding small molecules, polyethylenglycol, hydroxyethyl starch.

In a 34^{th} embodiment, in the recombinant Factor VIII protein of embodiment 33, said heterologous fusion partner is inserted directly C-terminal to said processing sequence. In a 35^{th} embodiment, in the recombinant Factor VIII protein of any of embodiments 33 or 34, said heterologous fusion partner is inserted C-terminal to the C2-domain.

In a 36^{th} embodiment, the recombinant Factor VIII protein of any one of the preceding embodiments further comprises a third thrombin cleavage site between the A1 and A2 domain.

The invention further provides, as a 37^{th} embodiment, a nucleic acid encoding a recombinant Factor VIII protein of any one of the preceding embodiments, wherein said polynucleotide optionally is an expression vector suitable for expression of said recombinant Factor VIII protein in a mammalian cell, e.g., a human cell.

The invention further provides, as a 38^{th} embodiment, a host cell comprising a nucleic acid of embodiment 37, wherein preferably the host cell is a mammalian cell comprising an expression vector suitable for expression of said recombinant Factor VIII protein in said cell. The cell can be a human cell selected from the group comprising a Hek293 cell line or a CAP cell line. Preferably, the cell is a CAP cell line.

The invention further provides, as a 39^{th} embodiment, a method for preparing a Factor VIII protein, comprising culturing the host cell of embodiment 38 under conditions suitable for expression of the Factor VIII protein and isolating the Factor VIII protein, wherein the method optionally comprises formulating the Factor VIII protein as a pharmaceutical composition.

In a 40^{th} embodiment, the invention provides a composition comprising recombinant Factor VIII protein of any one of embodiments 1-36, wherein the content of single chain protein Factor VIII protein of all Factor VIII protein is at least 90%.

The invention further provides, as a 41^{st} embodiment, a pharmaceutical composition comprising the recombinant Factor VIII protein of any of embodiments 1-36 or 40, the nucleic acid of embodiment 37, or the host cell of embodiment 38. The pharmaceutical composition may comprise a pharmaceutically acceptable solvent, e.g., water or a buffer, and/or pharmaceutically acceptable excipients. In a 42^{nd} embodiment, the pharmaceutical composition of embodiment 41, or a kit comprising the pharmaceutical composition of embodiment 41, further comprises an immunosuppressive agent, e.g., immunosuppressive agent selected from the group comprising methylprednisolone, prednisolone, dexamethason, cyclophosphamide, rituximab, and/or cyclosporin.

The invention further provides, as a 43^{rd} embodiment, a pharmaceutical composition of any of embodiments 41 or 42 for use in treatment of hemophilia A, wherein, optionally, the treatment is immune tolerance induction (ITI). In a 44^{th} embodiment, the pharmaceutical composition of any of embodiments 41-43 is for use in treating a patient with Hemophilia A selected from the group comprising a patient not previously treated with any Factor VIII protein, a patient previously treated with a Factor VIII protein, a patient who has an antibody response including an inhibitory antibody response to a Factor VIII protein, and a patient who has had an antibody response including an inhibitory antibody response to a Factor VIII protein who has been treated by ITI, or who has not been treated by ITI.

In a 45^{th} embodiment, the invention provides a vial, e.g., a prefilled or ready-to use syringe, comprising the pharmaceutical composition of any of embodiments 41-44.

In a 46^{th} embodiment, the invention provides a method of treatment, comprising administering an effective amount of the pharmaceutical composition of any of embodiments 41-44 to a patient in need thereof, e.g., a patient with hemophilia A, which may be selected from the patient groups defined herein.

### Figure legends

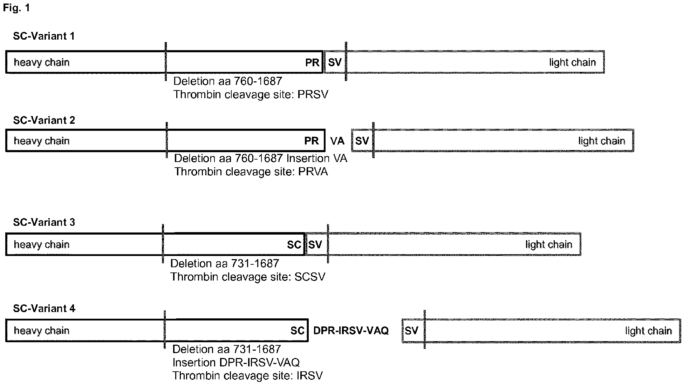

**Fig. 1** shows comparative single chain FVIII protein constructs in which the furin cleavage recognition site was deleted. Variants AC_SC-V1 (V1) and -V3 (V3) have a natural thrombin cleavage site (PR/SV or SC/SV, respectively), and there is only a single deletion of aa (amino acid/s) 760-1687 (V1) and aa 731-1687 (V3). Variants AC_SC-V2 (V2) and -V4 (V4), compared to V1 and V2, comprise a different thrombin cleavage site (PR/VA or IR/SV, respectively), wherein V2, based on V1, comprises a VA sequence which can be considered as being derived from the B-domain. V4, based on V3, comprises an insertion DPR-IRSV-VAQ at the site of the deletion.

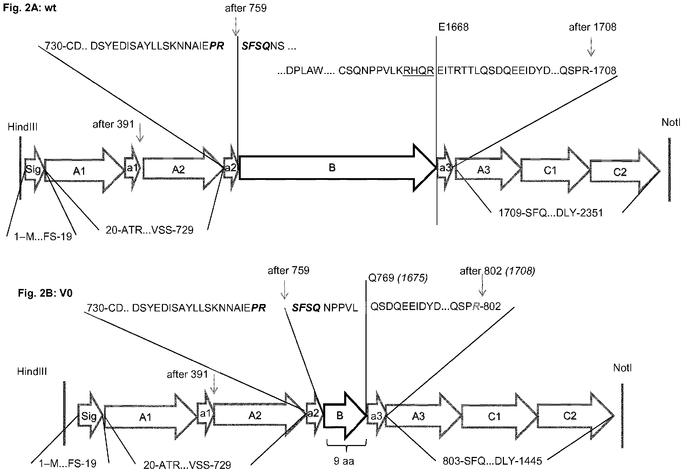

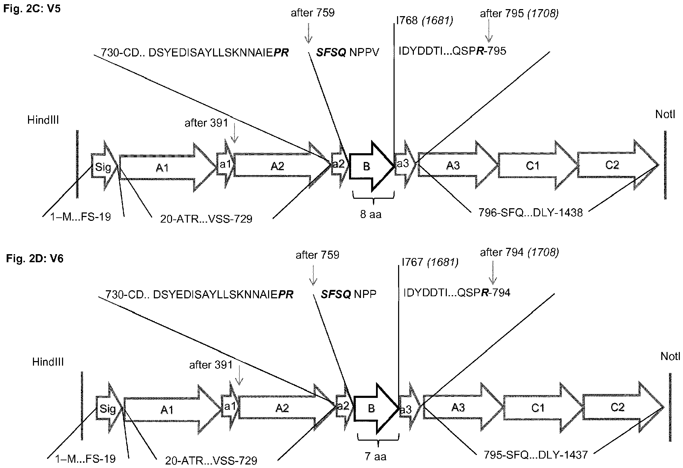

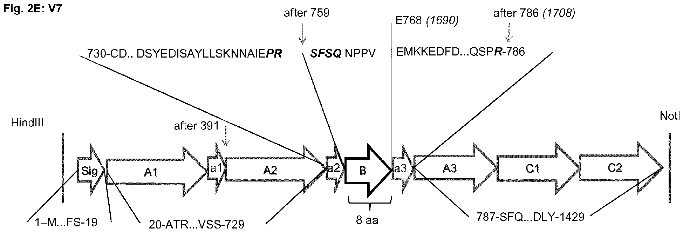

**Fig. 2** shows wt FVIII (A) and single chain FVIII protein constructs AC_SC-V0 (B), -V5 (C), - V6 (D), and -V7 (E) (or, short, V0, V5, V6, V7), which are based on the ReFacto AF® amino acid sequence AC-6rs-Ref SC, by introduction of two deletions. Arrows show thrombin cleavage sites. Sig is the signal peptide (19 aa). Bold and italic letters indicate the amino acids of the thrombin cleavage recognition site with cleavage after aa 759 (i.e., in the processing sequence in the constructs of the invention). The furin cleavage recognition site is underlined in the wildtype protein. The italic numbers in parenthesis relate to the amino acid numbers in the wild type sequence.

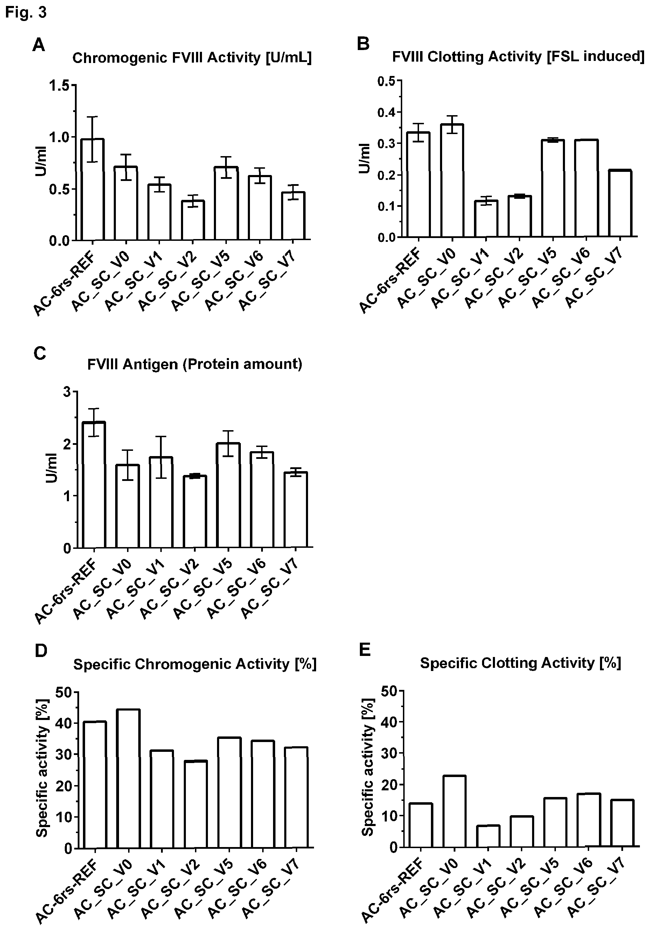

**Fig. 3** Comparison of unpurified single chain FVIII variants for their *in vitro* functionality. Cell culture supernatants of CAP-T cells expressing the double chain FVIII molecule AC-6rs-REF, and the single chain FVIII variants AC_SC-V0, -V1, -V2, -V5, -V6, -V7 were analysed for chromogenic FVIII activity (A), FVIII antigen levels indicating total FVIII protein amount (B) and FVIII clotting activity induced by Actin FSL (C). Specific activity was calculated as chromogenic FVIII activity to FVIII antigen ratio displayed in % (D). n=2.

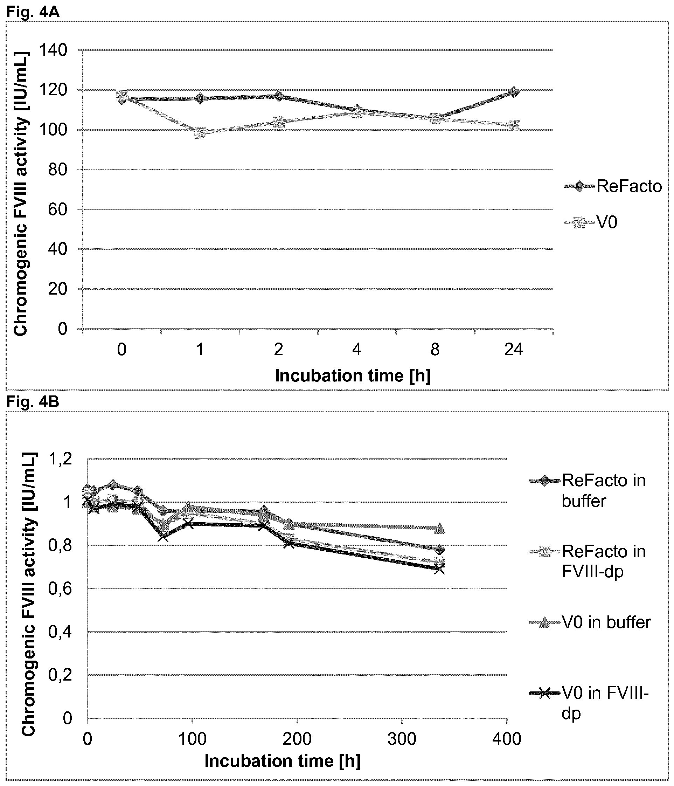

**Fig. 4** shows a comparison of stability (as determined by chromogenic activity) of V0 and ReFacto AF® *in vitro* over 24 h (A) and over 14 days (B). In A, stability is analysed in buffer and ReFacto AF® is shown with diamonds and V0 (AC-SC) with squares. In B, ReFacto AF® in FVIII formulation buffer is shown, with diamonds, and in FVIII-depleted plasma (FVIII-dp), with squares, and V0 in buffer, with triangles, and in FVIII-dp, with crosses.

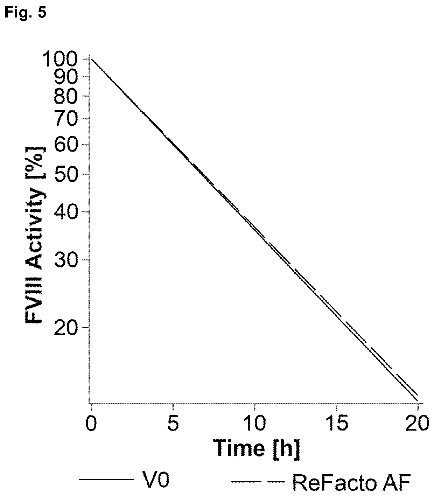

**Fig. 5** shows the regression curve of normalized activity (as determined by chromogenic activity) of V0 (continuous line) and ReFacto AF® (dashed line) as a result of a non-compartment analysis from an *in vivo* pharmacokinetic study in Hemophila A mice.

### Sequences

SEQ ID NO: 1 wild type Factor VIII
SEQ ID NO: 2 PRSFSQNPP minimal processing sequence
SEQ ID NO: 3 PRSFSQNPPV processing sequence
SEQ ID NO: 4 PRSFSQNPPVL processing sequence
SEQ ID NO: 5 PRSXSQNPPVL processing sequence
SEQ ID NO: 6 PRSFXQNPPVL processing sequence
SEQ ID NO: 7 PRSFSXNPPVL processing sequence
SEQ ID NO: 8 PRSFSQXPPVL processing sequence
SEQ ID NO: 9 QSDQEEIDYD, merging sequence, e.g., in V0
SEQ ID NO: 10 IDYDDTI merging sequence, e.g., in V5+V6
SEQ ID NO: 11 EMKKEDFD merging sequence, e.g., in V7
SEQ ID NO: 12 *Q1675 to R1708 von V0*, merging sequence, e.g., in V0
SEQ ID NO: 13 *I1681 to R1708 von V6*, merging sequence, e.g., in V6
SEQ ID NO: 14 *E1690 to R1708 von V7*, merging sequence, e.g., in V7
SEQ ID NO: 15 KRHQREITRTT sequence comprising furin cleavage recognition site deleted in proteins of the invention
SEQ ID NO: 16 V0 aa sequence
SEQ ID NO: 17 V1 aa sequence
SEQ ID NO: 18 V2 aa sequence
SEQ ID NO: 19 V3 aa sequence
SEQ ID NO: 20 V4 aa sequence
SEQ ID NO: 21 V5 aa sequence
SEQ ID NO: 22 V6 aa sequence
SEQ ID NO: 23 V7 aa sequence
SEQ ID NO: 24 V0 na sequence
SEQ ID NO: 25 V5 na sequence
SEQ ID NO: 26 V6 na sequence
SEQ ID NO: 27 V7 na sequence
SEQ ID NO: 28 AC-6rs-REF aa sequence
SEQ ID NO: 29 AC-6rs aa sequence
SEQ ID NO: 30 AC-6rs-REF na sequence
SEQ ID NO: 31 SVEMKKEDF merging sequence in V1
SEQ ID NO: 32 DSYEDISAYLLSKNNAIE***PR*** sequence N-terminal to processing sequence and including 2 aa of processing sequence, i.e., sequence N-terminal of thrombin cleavage site
SEQ ID NO: 33-38 partial FVIII sequences

### Detailed description of the invention

In a first embodiment, the invention provides a recombinant Factor VIII protein comprising, in a single chain, a heavy chain portion comprising an A1 and an A2 domain and a light chain portion comprising an A3, C1 and C2 domain of Factor VIII, wherein,
a) in said recombinant Factor VIII protein, 894 amino acids corresponding to consecutive amino acids between F761 and P1659 of wild type Factor VIII as defined in SEQ ID NO: 1 are deleted, leading to a first deletion;
b) said recombinant Factor VIII protein comprises, spanning the site of the first deletion, a processing sequence comprising SEQ ID NO: 2 or a sequence having at most one amino acid substitution in SEQ ID NO: 2, wherein said processing sequence comprises a first thrombin cleavage site;
c) in said recombinant Factor VIII protein, at least the amino acids corresponding to amino acids R1664 to R1667 of wild type Factor VIII are deleted, leading to a second deletion; and
d) said recombinant Factor VIII protein comprises, C-terminal to the second deletion and N-terminal of the A3 domain, a second thrombin cleavage site.

The inventors have construed new FVIII single chain variants in which the region comprising a deletion of the B-domain is shorter than in other single-chain FVIII variants. In particular, by retaining an internal fragment of the B-domain, the inventors have managed to form a processing sequence (see SEQ ID NO. 2) which is closer to the wild type processing sequence. Preferably, part of the processing sequence corresponds to a truncated B-domain, thus the processing sequence is embedded into a sequence derived from wild type FVIII. As a consequence of these findings, the resulting FVIII proteins show a high level of expression, a low profile of fragments and side products, and in particular a high specific activity as evidenced by different biological activity assays. Furthermore, the inventors have found certain amniocyte cell lines to be particularly well suited for high-level expression of functional molecules.

Further advantages and preferred embodiments are explained elsewhere in this description.

Side-by-side comparisons with either double-chain FVIII (using the Moroctocog Alfa sequence, see Examples) or single-chain variants of the state of the art (variants V1 and V2, see Examples), show high expression levels combined with improved specific activity. The FVIII proteins according to the invention produce also much better results than other single chain variants designed and tested (V3, V4).

The skilled person understands the term FVIII (or Factor VIII) and is aware of the structure and biological functions of wild type FVIII and typical variants thereof. Apart from the sequences specified above, the FVIII protein of the invention may be designed as deemed appropriate and advantageous by the skilled person. In particular, the Factor VIII protein of the invention should typically comprise all necessary portions and domains known to be important for biological function. For example, preferably, the FVIII protein further comprises domains corresponding to, substantially corresponding to, and/or functionally corresponding to the A and C domains of wild type FVIII. It may further comprise additional portions and domains. For example, preferably, the FVIII protein further comprises an a1 domain between the A1 and the A2 domains and an a2 domain C-terminal to the A2 domain, wherein the processing sequence is located at the C-terminal end of the a2 domain. Part of the processing sequence corresponds to a truncated B-domain. C-terminal to said processing sequence, the FVIII protein comprises at least a truncated a3 domain, which may comprise a merging sequence as defined herein. Before processing, the Factor VIII protein of the invention may also comprise a signal sequence. Any or all of said domains may be wildtype (wt) FVIII domains, or they may differ from the wildtype domains, e.g., as known in the state of the art or deemed appropriate by the skilled person. The domains are preferably contained in the protein in that order, i.e., from N-terminus to C-terminus of the protein.

A FVIII protein according to the present invention shall have at least one biological activity or function of a wt FVIII protein, in particular with regard to the function in coagulation. The FVIII protein should be cleavable by thrombin, leading to activation. Preferably, said thrombin recognition and/or thrombin cleavage sites correspond to or substantially correspond to those of wild type FVIII. It is then capable of forming a complex with the activated coagulation Factor IXa and coagulation Factor X, and the light chain is capable of binding to a phospholipid bilayer, e.g., the cell membrane of (activated) platelets.

The biological activity of FVIII can be determined by analyzing the chromogenic or the coagulant activity of the protein, as described herein. Typically, the chromogenic activity is taken as a measure of biological activity.

The other parts of the FVIII protein of the invention can be designed as desired by the skilled person, but preferably maintaining a high FVIII biological activity. As shown in the Examples, the invention allows to generate a FVIII protein with a high biological activity, as measured e.g. by the chromogenic activity. Therefore, preferably the FVIII protein according to the invention has a chromogenic activity which is at least comparable to the activity of the wt protein, i.e., it has at least 50% of the chromogenic activity of the wt protein (SEQ ID NO: 1). Preferably, the FVIII protein according to the invention has at least 80%, at least 100 % or more than 100% of the chromogenic activity of the wt protein. Preferably, the chromogenic activity also is at least 80%, at least 90%, at least 100% or more than 100% of the chromogenic activity of ReFacto AF® (international non-proprietary name: Moroctocog Alfa), a commercially available B-domain deleted FVIII (Pfizer).

As defined in a), in the FVIII of the invention, 894 amino acids corresponding to consecutive amino acids between F761 and P1659 of wild type Factor VIII as defined in SEQ ID NO: 1 are deleted in the Factor VIII protein of the invention, leading to a first deletion. In certain embodiments, in particular, starting from a numbering of amino acids in FVIII without deletions or insertions, the term "corresponding to" should be understood to mean "identical to".

For specific amino acids which may be mutated compared to the wt, an amino acid corresponding to the wild type aa is determined by an alignment e.g. using EMBOSS Needle (based on the Needleman-Wunsch algorithm; settings: MATRIX: "BLOSUM62", GAP OPEN: "20", GAP EXTEND:"0.5", END GAP PENALTY: "false", END GAP OPEN: "10", END GAP EXTEND: "0.5").

In order to assess sequence identities of two polypeptides, such an alignment may be performed in a two-step approach: I. A global protein alignment is performed using EMBOSS Needle (settings: MATRIX: "BLOSUM62", GAP OPEN: "20", GAP EXTEND:"0.5", END GAP PENALTY: "false", END GAP OPEN: "10", END GAP EXTEND: "0.5") to identify a particular region having the highest similarity. II. The exact sequence identity is defined by a second alignment using EMBOSS Needle (settings: MATRIX: "BLOSUM62", GAP OPEN: "20", GAP EXTEND:"0.5", END GAP PENALTY: "false", END GAP OPEN: "10", END GAP EXTEND: "0.5") comparing the fully overlapping polypeptide sequences identified in (I) while excluding non-paired amino acids.

"between" excludes the recited amino acids, e.g., it means that the recited amino acids are maintained. "deletion" or "deleted" does not necessitate that the protein was actually prepared by deleting amino acids previously present in a predecessor molecule, but it merely defines that the amino acids are absent, independent from the preparation of the molecule. For example, the protein can be produced based on nucleic acids prepared by *de novo* synthesis or by genetic engineering techniques.

As defined in b), the recombinant Factor VIII protein comprises, spanning the site of the first deletion, a processing sequence comprising SEQ ID NO: 2 (PRSFSQNPP) or a sequence having at most one amino acid substitution in SEQ ID NO: 2, wherein said processing sequence comprises a first thrombin cleavage site. Accordingly, at least one amino acid of the processing sequence corresponds to an amino acid C-terminal to the deletion and at least one amino acid of the processing sequence corresponds to an amino acid N-terminal to the deletion. The processing sequence comprises SEQ ID NO: 2 or a sequence having at most one amino acid substitution in SEQ ID NO: 2, i.e., the processing sequence can be longer. In particular, the processing sequence is selected from the group comprising SEQ ID NO: 2, 3, 4, 5, 6, 7 or 8. The inventors have found that a processing sequence of the invention enables a particularly good cleavage by thrombin.

Typically, the processing sequence is not longer than SEQ ID NO: 4. The processing sequence may be directly C-terminal to sequences from the a2 domain, e.g., wt a2 domain sequences. The first N-terminal two amino acids of the processing sequence may already belong to the a2 domain. Preferably, the amino acid directly N-terminal to the processing sequence is E.

One amino acid in SEQ ID NO: 2 can be substituted, e.g., to reduce immunogenicity. Optionally, the F, the S C-terminal to the F, the Q or the N are substituted.

The processing sequence may be SEQ ID NO: 2 or a sequence having at most one amino acid substitution in said sequence, wherein, optionally, the F, the S C-terminal to the F, the Q or the N are substituted. For example, the FVIII proteins of the invention V0, V5, V6 and V7 comprise SEQ ID NO: 2. The processing sequence of V6 consists of SEQ ID NO: 2.

The processing sequence may alternatively be SEQ ID NO: 3 (PRSFSQNPPV) or a sequence having at most one amino acid substitution in said sequence, wherein, optionally, the F, the S C-terminal to the F, the Q or the N are substituted. For example, the FVIII proteins of the invention V0, V5, and V7 comprise SEQ ID NO: 3. The processing sequence of V5 and V7 consists of SEQ ID NO: 3.

The processing sequence may alternatively be SEQ ID NO: 4 (PRSFSQNPPVL) or a sequence having at most one amino acid substitution in said sequence, wherein, optionally, the F, the S C-terminal to the F, the Q or the N are substituted. For example, the present inventors have shown that an L at the C-terminus of the processing sequence, as in SEQ ID NO: 4, 5, 6, 7 or 8, endows the FVIII with particularly good activity. The processing sequence of the FVIII protein of the invention V0, which has been found to be particularly advantageous, consists of SEQ ID NO: 4, which is a specific embodiment of SEQ ID NO: 5-8.

The alternative processing sequences SEQ ID NO: 5 (PRSXSQNPPVL), SEQ ID NO: 6 (PRSFXQNPPVL), SEQ ID NO: 7 (PRSFSXNPPVL) and SEQ ID NO: 8 (PRSFSQXPPVL) are variants in which X can be any naturally occurring amino acid. Optionally, X is a conservative substitution compared to the corresponding amino acid in SEQ ID NO: 4, i.e. a hydrophobic amino acid is substituted by a hydrophobic amino acid, a hydrophilic amino acid is substituted by a hydrophilic amino acid, an aromatic amino acid by an aromatic amino acid, an acid amino acid by an acid amino acid and a basic amino acid by a basic amino acid.

As defined in c), in the FVIII protein of the invention, the amino acids corresponding to amino acids R1664 to R1667 of wild type Factor VIII are deleted, leading to a second deletion. These amino acid correspond to the furin cleavage recognition site of wt FVIII. Accordingly, the protein is essentially not cleaved by furin. In a composition, at least 80%, optionally, at least 90% or at least 95% of the FVIII protein of the invention are present in a single chain form.

As defined in d), the recombinant Factor VIII protein of the invention comprises, C-terminal to the second deletion and N-terminal of the A3 domain, a second thrombin cleavage site. Accordingly, upon activation, the part of the FVIII protein between the thrombin cleavage site in the processing sequence and the second thrombin cleavage site are excised from the activated FVIII protein.

As a second embodiment, the invention also provides a recombinant Factor VIII protein comprising, in a single chain, a heavy chain portion comprising an A1 and an A2 domain and a light chain portion comprising an A3, C1 and C2 domain of Factor VIII, wherein,
a) said recombinant Factor VIII protein comprises a processing sequence comprising SEQ ID NO: 2 or a sequence having at most one amino acid substitution in SEQ ID NO: 2, wherein said processing sequence comprises a first thrombin cleavage site;
b) optionally, directly C-terminal to said processing sequence, said Factor VIII protein comprises a heterologous sequence;
c) directly C-terminal to said processing sequence, or, if present, directly C-terminal to said heterologous sequence, said Factor VIII protein comprises a merging sequence having at least 90% sequence identity to a sequence selected from the group consisting of SEQ ID NO: 9 (QSDQEEIDYD), SEQ ID NO: 10 (IDYDDTI) and SEQ ID NO: 11 (EMKKEDFD); and
d) said recombinant Factor VIII protein comprises, C-terminal to SEQ ID NO: 9-11, a second thrombin cleavage site.

Said Factor VIII protein optionally is a Factor VIII protein as described above as the first embodiment. In any case, the definitions provided herein apply to both embodiments.

As described in b), optionally, directly C-terminal to the processing sequence, said Factor VIII protein comprises a heterologous sequence. A heterologous sequence does not occur in the wild type protein at the same relative location, and it optionally does not consist of the same number of amino acids as a sequence deleted from the protein of the invention. Preferably, a heterologous sequence comprises a non-FVIII sequence of at least 10, optionally, at least 20, at least 30 or at least 40 amino acids that do not occur in wild type FVIII, and, preferably, has less than 30% sequence identity to any wildtype FVIII fragment, optionally, less than 25% sequence identity to any wildtype FVIII fragment. In particular, it may have less than 20% sequence identity to any wt FVIII sequence from the B-domain or the a3 domain. A heterologous sequence may however comprise subsequences which are found in FVIII. Optionally, at least 60% of the heterologous sequence are non-FVIII sequences.

As described in c), directly C-terminal to the processing sequence, or, if present, directly C-terminal to said heterologous sequence, said Factor VIII protein comprises a merging sequence having at least 90% sequence identity to a sequence selected from the group consisting of SEQ ID NO: 9 (QSDQEEIDYD), SEQ ID NO: 10 (IDYDDTI) and SEQ ID NO: 11 (EMKKEDFD). The term "merging sequence" illustrates that the sequence is merging into the final sequence together with the processing sequence, and, optionally, directly C-terminal to said sequence, which can be effected through a deletion in wt FVIII. The merging sequence may also be designated "a3 derived" sequence, which illustrates the origin of the sequence. It does not necessarily mean that the sequence corresponds to all a3 sequences comprised in the recombinant Factor VIII protein of the invention. Preferably, there are no a3-derived amino acids N-terminal to the defined merging sequence, but, in particular for SEQ ID NO: 9-11, there may be a3-derived amino acids C-terminal to said defined sequence, e.g., as defined in SEQ ID NO: 12, 13 or 14.

Preferably, the Factor VIII protein may comprise a merging sequence selected from the group consisting of SEQ ID NO: 9, 10 or 11. The inventors could show that it is advantageous if the FVIII protein comprises a longer sequence corresponding to parts of the a3 domain which are C-terminal of SEQ ID NO: 9, preferably resulting in the sequence of SEQ ID NO: 12. The same applies to SEQ ID NO: 10, preferably resulting in the sequence of SEQ ID NO: 13 and to SEQ ID NO: 11, preferably resulting in the sequence of SEQ ID NO: 14. Accordingly, preferably, the recombinant Factor VIII protein comprises SEQ ID NO: 9. For example the V0 protein comprises SEQ ID NO: 9. It also comprises SEQ ID NO: 10 and SEQ ID NO: 11, which are derived from the a3-region (at least partly) C-terminal to SEQ ID NO: 9. V5 and V6 comprise SEQ ID NO: 10 and V7 comprises SEQ ID NO: 11.

The Factor VIII proteins of the invention can also be defined insofar as amino acids corresponding to
i) F761 and S1656;
ii) S762 and Q1657;
iii) Q763 and N1658; or
iv) N764 and P1659
of wild type Factor VIII as defined in SEQ ID NO: 1 are adjacent to each other, wherein said amino acids are part of the sequence defined in SEQ ID NO: 2. This is due to the first deletion as defined in the first embodiment. The skilled person will note that this particular construction leads to maintenance of amino acids identical without the deletion and with the deletion. Thus, the structure of the protein may be less affected by the deletion than with other deletions of the B-domain known in the state of the art. "adjacent" in the context of the invention means the same as "immediately adjacent", and relates to the secondary structure of proteins.

In one embodiment of the invention, wherein no heterologous sequence is inserted directly C-terminal to the processing sequence, it is further preferred that the processing sequence is directly N-terminal to an amino acid corresponding to Q1675, 11681 or E1690 of wild type Factor VIII. For example, this occurs for Q1675 in V0, for I1681 in V5 and V6 and for E1690 in V7.

In a Factor VIII protein of the invention, the 7 N-terminal amino acids of the a3-domain are preferably absent (i.e., deleted), i.e., the a3-domain is partially deleted or truncated.

In a Factor VIII protein of the invention, the amino acids corresponding to amino acids K1663 to L1674 of wild type Factor VIII may be deleted, leading to a second deletion. This is the case, e.g., in V0 and V5-V7.

In FVIII proteins of the invention, due to the second deletion, amino acids corresponding to amino acids V1661 and L1674 of wild type Factor VIII as defined in SEQ ID NO: 1 may be adjacent to each other, or amino acids corresponding to amino acids L1662 and Q1675 of wild type Factor VIII may be adjacent to each other. V0 is an example of a protein for which this applies. In case a heterologous sequence is inserted directly C-terminal to the processing sequence, the amino acids corresponding to amino acids L1662 and Q1675 of wild type Factor VIII are not adjacent to each other.

In other FVIII proteins of the invention, e.g., V5, due to the second deletion, amino acids corresponding to amino acids V1661 and 11681 of wild type Factor VIII as defined in SEQ ID NO: 1 are adjacent to each other.

In other FVIII proteins of the invention, e.g., V6, due to the second deletion, amino acids corresponding to amino acids P1660 and I1681 of wild type Factor VIII as defined in SEQ ID NO: 1 are adjacent to each other.

In other FVIII proteins of the invention, e.g., V7, due to the second deletion, amino acids corresponding to amino acids V1661 and E1690 of wild type Factor VIII as defined in SEQ ID NO: 1 are adjacent to each other.

The recombinant Factor VIII protein of the invention does not comprise a furin cleavage recognition site. Preferably, it also does not comprise a sequence having more than 75% sequence identity to the furin cleavage recognition site RHQR between the processing sequence and the merging sequence. A deletion of the furin cleavage recognition site has been found to be superior to other mutations, e.g., substitutions. Optionally, the recombinant Factor VIII protein of the invention does not comprise a sequence having more than 50% sequence identity to the furin cleavage recognition site RHQR between the processing sequence and the merging sequence.

Preferably, other amino acids in the vicinity of the furin cleavage recognition site are also deleted. Accordingly, preferably, the FVIII proteins of the invention do not comprise a sequence having more than 30% sequence identity to SEQ ID NO: 15 (KRHQREITRTT, i.e. aa K1663-T1673 of SEQ ID NO: 1), which comprises the furin cleavage recognition site. Optionally, they do not comprise a sequence having more than 40% sequence identity to SEQ ID NO: 15.

As the furin cleavage recognition site is not present, the protein is essentially not cleaved by furin, and the content of single chain protein Factor VIII protein of all Factor VIII protein is at least 90%.

Particularly good results regarding expression and activity have been found for recombinant Factor VIII proteins of the invention, comprising a processing sequence and, C-terminal to said processing sequence, a merging sequence, wherein the processing sequence is selected from the group comprising SEQ ID NO: 2, 3, 4, 5, 6, 7 or 8, and the merging sequence is selected from the group comprising SEQ ID NO: 12, 13 or 14.

Therefore, the invention also provides recombinant Factor VIII proteins comprising a processing sequence and, C-terminal to said processing sequence, a merging sequence, wherein the processing sequence is selected from the group comprising SEQ ID NO: 2, 3, 4, 5, 6, 7 or 8, and the merging sequence is selected from the group comprising SEQ ID NO: 12, 13 or 14.

For example, the processing sequence may be SEQ ID NO: 4 and the merging sequence SEQ ID NO: 12, as, e.g., in construct V0. The processing sequence may also be SEQ ID NO: 5 and the merging sequence SEQ ID NO: 12. The processing sequence may be SEQ ID NO: 6 and the merging sequence SEQ ID NO: 12. The processing sequence may be SEQ ID NO: 7 and the merging sequence SEQ ID NO: 12. The processing sequence may be SEQ ID NO: 8 and the merging sequence SEQ ID NO: 12. The processing sequence may be SEQ ID NO: 3 and the merging sequence SEQ ID NO: 13, as, e.g., in V5. The processing sequence may be SEQ ID NO: 2 and the merging sequence SEQ ID NO: 13, as, e.g., in V6. The processing sequence may be SEQ ID NO: 3 and the merging sequence SEQ ID NO: 14, as, e.g., in V7. Optionally, said merging sequence is directly C-terminal to said processing sequence. Alternatively, a heterologous sequence may be inserted between the processing sequence and the merging sequence, e.g., as defined for the fusion partners below.

The recombinant Factor VIII protein of the invention typically further comprises a third thrombin cleavage site between the A1 and A2 domain. It may also comprise further thrombin cleavage sites, as long as the biological function is maintained, but this is not required.

Preferred FVIII proteins of the invention comprise the amino acid sequence of the mature protein (i.e., without signal sequence) of any of SEQ ID NO: 16 (V0), 21 (V5), 22 (V6) or 23 (V7), preferably, SEQ ID NO: 16, or a fusion protein of any of these proteins.

Thus, the invention provides recombinant Factor VIII molecules, comprising an amino acid sequence according to SEQ ID NO: 16, SEQ ID NO: 21, SEQ ID NO: 22, or SEQ ID NO: 23 (each without the signal sequence), or which are fusion proteins comprising at least one of these sequences. A protein comprising the amino acid sequence according to SEQ ID NO: 16 (without the signal sequence) has been shown to have particularly good characteristics. The signal sequence corresponds to amino acids 1-19 of the respective proteins. In the mature protein, which is typically used, in particular, for pharmaceutical purposes, the signal sequence is normally missing. However, it is also possible that the signal sequence is included in the protein of the invention.

A fusion partner may be employed to extend the *in vivo* plasma half-life of the FVIII protein of the invention. In one embodiment, the recombinant Factor VIII protein of the invention is a fusion protein with at least one heterologous fusion partner, preferably with a fusion partner extending the in vivo plasma half-life of the FVIII protein. The fusion partner may e.g. be selected from the group comprising an Fc region, albumin, an albumin binding sequence, PAS polypeptides, HAP polypeptides, the C-terminal peptide of the beta subunit of chorionic gonadotropin, albumin-binding small molecules, and combinations thereof. The FVIII protein may alternatively or additionally be covalently linked to non-protein fusion partners such as PEG (polyethylenglycol) and/or HES (hydroxyethyl starch). PAS polypeptides or PAS sequences are polypeptides comprising an amino acid sequence comprising mainly alanine and serine residues or comprising mainly alanine, proline and serine residues, the PAS sequences forming a random coil conformation under physiological conditions, as defined in WO 2015/023894. HAP polypeptides or sequences are homo-amino acid polymer (HAP), comprising e.g., repetitive sequences of Glycine or Glycine and Serine, as defined in WO 2015/023894. Potential fusions, fusion partners and combinations thereof are described in more detail e.g., in WO 2015/023894.

Optionally, for certain therapeutic applications, the recombinant FVIII protein may be fused to an Fc region. A fusion to an Fc region may be used to extend the half-life and reduce immunogenicity.

The inventors have found that said heterologous fusion partner may advantageously be inserted directly C-terminal to said processing sequence and/or C-terminal to the C2-domain. These locations have been found by the inventors to be advantageous for fusion, while maintaining good biological activity of the FVIII protein. Optionally, the fusion protein further comprises at least one linker.

The protein may further be glycosylated and/or sulfated. Preferably, post-translational modifications such as glycosylation and/or sulfation of the protein occur in a human cell. A particularly suitable profile of post-translational modifications can be achieved using CAP cells, in particular CAP-T cells or CAP-Go cells (WO 2001/36615; WO 2007/056994; WO 2010/094280; WO 2016/110302). CAP cells, available from Cevec Pharmaceuticals GmbH (Cologne, Germany), originate from human amniocytes as they were isolated trans-abdominally during routine amniocentesis. Obtained amniocytes were transformed with adenoviral functions (E1A, E1B, and pIX functions) and subsequently adapted to growth in suspension in serum-free medium.

wt FVIII typically is bound by vWF. vWF can shield against degradation and may have a positive impact on immune tolerance. Therefore, in certain embodiments of the invention, the protein should be capable of association with vWF. For example, the binding potency of the FVIII protein of the invention to vWF is 10%-100%, 10%-90%, 20-80%, 30-70%, 40-60% or 50-60% of the binding potency of ReFacto AF® to vWF, which can be determined by an ELISA-based method. vWF binding is mediated in particular by amino acid positions Y1683 and Y1699. These should not be mutated if vWF binding is desired.

In certain cases, it may be the case or even desirable that the FVIII protein is not capable of association with vWF. This may, for example, be the case if stabilization is mediated by different means than vWF binding. To avoid vWF binding, e.g. amino acids Y1683 and/or Y1699 may be mutated, or vWF binding may be sterically hindered by a different binding partner.

Advantageously, the FVIII protein according to the invention is capable of being sufficiently stable for pharmaceutical use. The inventors could show that stability of FVIII proteins of the invention *in vitro* and *in vivo* is comparable to stability of ReFacto AF®, see Examples. Therefore, the protein of the invention is preferably sufficiently stable in human plasma *in vitro* and/or *in vivo,* particularly in vivo. Preferably, *in vivo*, the half-life of the FVIII protein of the invention in human plasma (in a patient without inhibitors) is about at least 6 hours, preferably, at least 12 hours, at least 18 hours, at least 24 hours, or at least 30 hours. As defined herein, the FVIII protein may be a FVIII protein without fusion partner, or it may be a fusion protein as defined herein. However, as shown in the Examples, the specified half-life can already be obtained without fusion partners. In case of the presence of one or more fusion partners, the half-life of the FVIII protein may be the same, or even longer.

The invention also provides a nucleic acid encoding a recombinant Factor VIII protein of the invention. Said polynucleotide may be an expression vector, e.g., suitable for expression of said recombinant Factor VIII protein in a mammalian cell, such as a human cell.

The nucleic acid preferably encodes the FVIII with an N-terminal signal sequence, e.g., the 19 aa signal sequence of SEQ ID NO: 1. Preferred nucleic acids of the invention encode SEQ ID NO: 16 and 21-23 (V0, V5, V6, V7), or, optionally, a fusion protein thereof. They may be SEQ ID NO: 24-27. The nucleic acids of the invention may be DNA molecules or RNA molecules. The nucleic acids may be optimized for expression in the host cell, e.g., in a human cell.

The expression vector comprises the sequence encoding the FVIII protein, preferably, in codon-optimized form, under the functional control of a suitable promoter, which may be a constitutive or an inducible promoter. The promoter may be a promoter not associated with expression of FVIII in nature, e.g., EF-1alpha or a heterologous promoter, e.g., CMV or SV40. It may further comprise pro- and/or eukaryotic selection markers, such as ampicillin resistance and dihydrofolate reductase (dhfr), and origins of replication, e.g., an SV40 origin and/or a pBR322 origin. "codon-optimized" means optimized for expression in the host cell, preferably, for expression in a human host cell.

Alternatively, the nucleic acid may be a vector suitable for gene therapy, e.g., for gene therapy of a human patient. Vectors suitable for gene therapy are known in the art, e.g., virus-based vectors e.g., based on adenovirus or adeno-associated virus (AAV) or based on retrovirus, such as lentiviral vectors etc. or non virus-based vectors such as but not limited to small plasmids and minicircles or transposon-based vectors. An AAV-based vector of the invention may e.g., be packaged in AAV particles for gene therapy of Hemophilia A patients.

The invention also provides a host cell comprising a nucleic acid of the invention. The host cell may be a bacterial cell, a plant cell, a fungal cell, a yeast cell or an animal cell. Preferably, the host cell is an animal cell, in particular, a mammalian cell comprising an expression vector suitable for expression of said recombinant Factor VIII protein in said cell. The host cell preferably is a human cell comprising an expression vector suitable for expression of said recombinant Factor VIII protein in said human cell. The cell may be transiently or stably transfected with the nucleic acid of the invention. The cell may be a cell line, a primary cell or a stem cell. For production of the protein, the cell typically is a cell line such as a HEK cell, such as a HEK-293 cell, a CHO cell, a BHK cell, a human embryonic retinal cell such as Crucell's Per.C6 or a human amniocyte cell such as CAP. For treatment of human patients with the protein, the host cell preferably is a human cell, e.g., a HEK293 cell line or a CAP cell line (e.g. a CAP-T cell or a CAP-Go cell). The inventors have found that in a CAP cell line, a particularly high single chain content of FVIII protein of the invention is produced. Among the CAP cells, CAP-T cells are preferred for transient expression, while CAP-Go cells may be used for creation of stable cell lines conveying an advantageous glycosylation profile to the FVIII molecule.

The cell may be an autologous cell of a Hemophilia A patient suitable for producing FVIII in the patient after transfection and reintroduction into the patient's body. The cell may be a stem cell, e.g., a hematopoietic stem cell, but preferably it is not an embryonic stem cell, in particular when the patient is a human. The cell may also be hepatocyte, a liver sinusoidal endothelial cell or a thrombocyte.

Cell lines expressing the protein of the invention may also be used in a method of preparing the protein of the invention, comprising cultivating said cells under conditions suitable for expression of the FVIII protein and purifying said protein, e.g., using a plurality of methods known to the skilled person, e.g., as described herein. Such purification methods may comprise standard harvesting procedures for cell removal, e.g. centrifugation, followed by chromatography steps, e.g. affinity chromatography, and methods for exchanging the FVIII proteins into a suitable buffer. The invention thus also provides a method for preparing a Factor VIII protein, comprising culturing the host cell of the invention under conditions suitable for expression of the Factor VIII protein and isolating the Factor VIII protein, wherein the method optionally comprises formulating the Factor VIII protein as a pharmaceutical composition.

The invention thus provides a pharmaceutical composition comprising the recombinant Factor VIII protein of the invention, the nucleic acid of the invention or the host cell of the invention. Such pharmaceutical compositions may comprise suitable excipients, e.g., a buffer, a stabilizing agent, a bulking agent, a preservative, another (e.g., recombinant) protein or combinations thereof. In the context of the invention, if not explicitly stated otherwise, "a" is understood to mean one or more. A suitable buffer for formulation may e.g. contain 205 mM NaCl, 5.3 mM CaCl₂, 6.7 mM L-Histidine, 1.3 % Sucrose and 0.013 % Tween 20 in distilled water and have a pH of 7.0 (FVIII formulation buffer). Said buffer is used in the experiments described herein if not otherwise stated. Formulations of FVIII may be sterile, e.g., sterile filtered, in particular for *in vivo* use.

The pharmaceutical composition may be formulated as desired appropriate by the skilled person, e.g., for intravenous (i.v.) or subcutaneous application, intraperitoneal or intramuscular application. Generally, it is for administration as slow i.v. push bolus injection. Continuous infusion is indicated e.g., for patients requiring admission for severe bleeds or surgical procedures. Oral application, which may contribute to tolerance induction, is also possible, e.g., after expression in plants. The pharmaceutical composition may be for slow release.

Pharmaceutical compositions comprising FVIII can be lyophilized. Dosages and treatment schemes may be chosen as appropriate, e.g., for prophylaxis of bleeding or with intermittent, on-demand therapy for bleeding events. Decisions on dosing may be made by the physician. Dosing depends on the patent, e.g., weight, FVIII status etc. For example, the FVIII of the invention may be administered in dosages of 0.5 to 250 IU/kg body weight every 0.5 to 6 days intravenously depending on the severity of the disease, typically, 0.5 to 200 IU/kg body weight. The invention also provides a pharmaceutical composition comprising the FVIII protein of the invention in combination with an immunosuppressive agent (e.g., methylprednisolone, prednisolone, dexamethasone, cyclophosphamide, rituximab, and/or cyclosporin), and/or it may be for administration at substantially the same time with (e.g. within minutes to 12 hours) with such an agent.

The pharmaceutical composition, e.g., comprising the protein of the invention, may be for use in treating a patient in need thereof, in particular, a Hemophilia A patient, e.g., a patient with acquired hemophilia involving an autoimmune response to FVIII or a congenital Hemophilia A patient. Mammals such as mice or dogs may be treated with the pharmaceutical composition of the invention, but the patient typically is a human patient.

The pharmaceutical composition of the invention may also be used for treatment of a patient previously treated with a recombinant and/or plasmatic Factor VIII protein. In a patient who has an antibody including an inhibitory antibody response to a recombinant and/or plasmatic Factor VIII protein, the pharmaceutical compositions may, e.g., be used for immune tolerance induction (ITI) treatment. The compositions of the invention may thus also be used for rescue ITI. The pharmaceutical compositions may also be advantageously used in a patient who has had an antibody response including an inhibitory antibody response to a recombinant and/or plasmatic Factor VIII protein, e.g., who has been treated by ITI. The pharmaceutical compositions may also be advantageously used in a patient who has had an antibody response including an inhibitory antibody response to a recombinant and/or plasmatic Factor VIII protein, who has not been treated by ITI.

The invention also provides a vial comprising the pharmaceutical composition of the invention, e.g., a syringe. The syringe may be a pre-filled syringe, e.g., a ready-to-use syringe.

All publications cited herein are fully incorporated herewith. The invention is further illustrated by the following examples, which are not to be understood as limiting the scope of the invention.

### Examples

### 1. Generation of single chain variants and determination of biological activity

During the development of a new recombinant hemophilia A therapeutic, the possibility of using a single chain FVIII molecule lacking the furin cleavage recognition site and thereby inhibiting enzymatic cleavage has been tested. Said single chain FVIII molecules may be favorable in terms of stability during purification and storage, but may also have benefits *in vivo* in terms of stability e.g. for subcutaneous administration and plasma half-life. Therefore, DNA plasmids encoding different B-domain truncated FVIII single chain variants each having individual deletions including the furin cleavage recognition site were designed and their functionality assessed using *in vitro* assays.

### Material and methods

### Preparation of constructs

As a basic FVIII sequence for cloning, a codon-optimized sequence of Refacto AF® was used, wherein for simplifying cloning, 6 restriction sites were added through silent mutations. Some of these restriction sites were again excluded due to codon-optimization. The basic sequence is AC-6rs-REF (SEQ ID NO: 28).

For the constructs encoding the FVIII of the invention and comparative constructs also analysed in this context, either the complete FVIII sequence or DNA regions encoding 550-600 aa from the FVIII a2 domain to the A3 domain were synthesized. The complete synthesized DNA was codon-optimized. The DNA fragments were 5' terminally flanked by an EcoRV restrictions site, and 3' terminally flanked by an EcoRI restriction site, and these restriction sites were also present in the basic FVIII sequence used. Restriction of the DNA inserts and the FVIII backbone plasmid allowed for targeted ligation and generation of FVIII single chain plasmids. Completely synthesized FVIII DNA was 5' terminally flanked by a HindIII restrictions site, and 3' terminally flanked by a Notl restriction site.

By transformation of *E.coli* K12 with said plasmids, expansion of transformed bacteria under ampicillin selection and plasmid preparation, large amounts of the plasmids could be prepared. Genetic engineering work was carried out by Thermo Fisher Scientific after design with VectorNTI Software (Thermo Fisher Scientific, Massachusetts, USA).

### Cultivation of CAP-T cells

CAP-T cells (Cevec Pharmaceuticals, Köln, Germany) were cultured in PEM medium supplemented with 4 mM GlutaMAX (Thermo Fisher Scientific, 35050038) and 5 µg/ml blasticidin (Thermo Fisher Scientific, R21001; complete PEM medium). In order to thaw the cells, the required amount of frozen vials were transferred to a 37 °C water bath. After thawing, each vial was transferred to 10 ml of chilled, complete PEM medium. The cell suspension was centrifuged at 150 x g for 5 minutes. During this washing step the dimethyl sulfoxide (DMSO) used for cryopreservation was removed. The pellet was resuspended in 15 ml warm, complete PEM medium and transferred to a 125 ml shaker flask. The cells were incubated at 37 °C in a humidified incubator with an atmosphere containing 5 % CO₂. The flasks were set on a shaking platform, rotating at 185 rpm with an orbit of 50 mm.

Subculturing of the cells was performed every 3 to 4 days. The fresh culture was set to 0.5x10⁶ cells/ml by transferring the required amount of cultured cell suspension to a new flask and adding complete PEM medium. In the case that the transferred cell suspension would exceed 20% of the total volume, the suspension was centrifuged at 150 x g for 5 minutes and the pellet was resuspended in fresh complete PEM medium. The volume of cell suspension per shaking flask was 20% of the total flask volume.

A minimum of three subcultures were performed after thawing before transfection experiments were performed.

### Protein expression in CAP-T cells by transient transfection

The CAP-T cells were transfected using the 4D-Nucleofector™ (Lonza, Basel, Switzerland). For each transfection 10x10⁶ CAP-T cells were centrifuged at 150 x g for 5 minutes in 15 ml conical tubes. The cells were resuspended in 95 µl supplemented SE Buffer, taking into account the volume of the pellet and the volume of the plasmid solution. Afterwards, 5 µg of the respective plasmid were added to the cell suspension followed by gentle mixing. The solution was transferred to 100 µl Nucleocuvettes. The used transfection program was ED-100. After the transfection, the cells from one Nucleocuvette were transferred to 125 ml shaker flasks, containing 12.5 ml complete PEM medium. The cells were cultivated for 4 days as described above. At day 4 the cells were harvested by centrifugation at 150 x g for 5 minutes. Larger protein amounts could be produced by combining 12.5 ml approaches as described above.

### Chromogenic FVIII Activity

The activity of FVIII was determined by a chromogenic assay. In this two-step assay, FIXa and FVIIIa activate FX in the first step. In the second step, the activated FX hydrolyses a chromogenic substrate, resulting in a color change, which can be measured at 405 nm. Due to the fact that calcium and phospholipids are present in optimal amounts and an excess of FIXa and FX is available, the activation rate of FX is only dependent on the amount of active FVIII in the sample.

The reagents for this chromogenic FVIII activity assay were taken from the Coatest® SP FVIII Kit. The kit contained phospholipids, calcium chloride (CaCl₂), trace amounts of thrombin, the substrate S-2765, a mixture of FIXa and FX and the thrombin inhibitor I-2581. The inhibitor was added, in order to prevent hydrolysis of the substrate by thrombin, which was built during the reaction. All dilutions were performed in distilled water or Tris-BSA (TBSA) Buffer, containing 25 mM Tris, 150 mM sodium chloride (NaCl) and 1 % Bovine serum albumin (BSA), set to pH 7.4. Each sample was diluted at least 1:2 with FVIII-depleted plasma. Further dilutions were performed using the TBSA Buffer.

The assay was performed using the BCS XP (Siemens Healthcare, Erlangen, Germany), a fully automated hemostasis analyzer. All reagents including water, TBSA Buffer and the samples were inserted into the analyzer. For each sample the analyzer mixed 34 µl calcium chloride, 20 µl TBSA Buffer, 10 µl sample, 40 µl water, 11 µl phospholipids and 56 µl FIXa-FX-mixture. This mixture was incubated for 300 seconds. Afterwards, 50 µl of S-2765 + I-2581 were added to the reaction. Upon addition of the substrate, the absorption at 405 nm was measured for 200 seconds.

In order to calculate the amount of active FVIII, the software of the analyzer evaluated the slope of the measured kinetic between 30 seconds and 190 seconds after starting the reaction. This result was correlated to a calibration curve, generated with a biological reference preparation (BRP) of FVIII. The activity of the BRP is indicated in IU/ml. However, IU/ml can be assumed equivalent to U/ml. The results were indicated as "% of normal". These results were converted to U/ml, as 100 % of normal FVIII activity are equivalent to 1 U FVIII activity per ml.

### Clotting Activity FSL

In addition to the two-stage chromogenic assay (see above), a one-stage clotting assay was also performed in order to determine the amount of active FVIII. During this assay, FVIII-depleted plasma, CaCl₂, the activator Actin FSL and the FVIII-containing sample are mixed in one step. The activator leads to the generation of FXIa, which activates FIX. FVIIIa, FIXa and FX built the tenase complex and FX becomes activated. Further activation of prothrombin and fibrinogen finally leads to the formation of a fibrin clot. The time needed to form the clot, the activated partial thromboplastin time (aPTT), is measured. The aPTT varies, depending on the amount of FVIII.

The clotting assay was performed using the BCS XP. TBSA Buffer, FVIII-depleted plasma, Actin FSL, CaCl₂ and the sample were inserted into the analyzer. The sample was diluted at least 1:2 with FVIII-depleted plasma. Further dilutions were performed using the TBSA Buffer. For each sample the analyzer mixed 45 µl TBSA Buffer, 5 µl sample, 50 µl FVIII-depleted plasma and 50 µl Actin FSL. The reaction was started by the addition of 50 µl CaCl₂. The analyzer measured the time needed for clot formation.

In order to calculate the amount of active FVIII, the software of the analyzer evaluated a baseline extinction at 405 nm at the beginning of the reaction. All of the following extinction values, within a time of 200 seconds, were analysed regarding their difference to the baseline extinction. The first time point exceeding a defined threshold was determined as the clotting time. This result was correlated to a calibration curve, generated with a BRP of FVIII.

### FVIII antigen ELISA

The amount of FVIII antigen was determined using the Asserachrom® VIII:Ag ELISA (Diagnostica Stago, Asnières sur Seine Cedex, France). In this sandwich ELISA, the applied FVIII is bound by mouse monoclonal anti-human FVIII F(ab')₂ fragments, which are coated to the plate by the manufacturer. The detection of the bound FVIII occurs via mouse monoclonal anti-human FVIII antibodies, which are coupled to a peroxidase. In the case that FVIII is present, the peroxidase-coupled antibody binds to FVIII and can be detected by the addition of a tetramethylbenzidine (TMB) solution. TMB turns from a clear to a blue-green solution upon reaction with peroxidase. After a short time, this reaction is stopped by the addition of sulfuric acid (H₂SO₄), which turns the solution yellow. The amount of bound FVIII correlates with the intensity of the yellow color, which can be measured at 450 nm. The final amounts of FVIII are calculated using a calibration curve generated by the measurement of at least five serial dilutions of a calibrator with a known antigen concentration.

The supplied calibrator and control were reconstituted with 500 µl of distilled water, 30 minutes before starting the ELISA. After this incubation time, the calibrator was diluted 1:10 in the supplied phosphate buffer. This represented the starting concentration. The calibrator was further serially diluted 1:2 up to a dilution of 1:64. As the concentration of the calibrator contained approximately 1 U/ml FVIII, depending on the batch, the starting concentration was equivalent to 0.1 U/ml FVIII whereas the last dilution contained approximately 0.0016 U/ml FVIII. The control was diluted 1:10 and 1:20 with the phosphate buffer. All samples were diluted with the phosphate buffer, depending on their previously determined activity (see above) with the aim to be in the middle of the calibration curve. After the dilution of FVIII samples, control and calibrator, 200 µl of each solution were applied per well in duplicates. In addition to that, two wells were filled with 200 µl of phosphate buffer as a blank control. The plate was incubated for 2 hours at room temperature covered with a film. During this time, the peroxidase-coupled anti-human FVIII antibodies were reconstituted with 8 ml phosphate buffer and incubated 30 minutes at room temperature. After the antigen immobilization, the wells were washed five times with the supplied washing solution, which was previously diluted 1:20 with distilled water. Immediately after the washing, 200 µl of the peroxidase-coupled anti-human FVIII antibodies were added to each well and incubated for 2 hours at room temperature covered by a film. Afterwards, the plate was washed five times as before. In order to reveal the amount of bound FVIII, 200 µl of TMB solution were added to each well and incubated for exactly 5 minutes at room temperature. This reaction was stopped by the addition of 50 µl 1 M H₂SO₄ to each well. After an incubation time of 15 minutes at room temperature, the absorbance of each well was measured at 450 nm using the POLARstar Omega plate reader (BMG LABTECH, Ortenberg, Germany).

The results of the ELISA were calculated using the MARS software (BMG Labtech). In a first step, all wells were blank corrected and the mean of the duplicates was calculated. Afterwards, a 4-parameter fit was applied, in order to calculate the concentrations from the calibration curve. According to this calibration curve the amount of FVIII antigen in each well was determined. In the last step, the values were corrected by the dilution factor, resulting in the FVIII antigen amount of each sample.

### Results and discussion

Different variants of single chain FVIII molecules (Fig. 1, 2) were generated and analysed. In all variants, the furin cleavage recognition site was deleted. Variants AC_SC-V1 (V1) and -V3 (V3) have a natural thrombin cleavage site (PR/SV or SC/SV, respectively), and there is only a single deletion of aa 760-1687 (V1) and aa 731-1687 (V3). Variants AC_SC-V2 (V2) and - V4 (V4), compared to V1 and V3, comprise a different thrombin cleavage site (PR/VA or IR/SV, respectively), wherein V2, based on V1, further comprises a VA sequence which can be considered as being derived from the B-domain. V4, based on V3, comprises an insertion DPR-IRSV-VAQ at the site of the deletion (Fig. 1). None of the constructs V1-V4 comprise a processing sequence as required by the present invention, e.g., including the sequence NPP in the context of the thrombin cleavage site of interest.

The inventors performed further experimentation by generating additional constructs, designated AC_SC-V0, -V5, -V6 and -V7 (or, short, V0, V5, V6 and V7), shown in Fig. 2. In a less straightforward manner, they moved from a single deletion of the B-domain to creating two deletions, which are bridged by retaining a short fragment of the B-domain. Thus, a processing sequence was generated, which seemed to be closer to the wild type sequence. The constructs are based on the Refacto AF® amino acid sequence AC-6rs-Ref, by introduction of two deletions, i.e. proteins of the invention comprising a processing sequence as defined herein. Thus, V0, V5, V6 and V7 represent FVIII proteins according to the present invention.

Western-Blots of V0-V7 show expression of all analysed constructs mainly as a single chain molecule (not shown), which is indicated by a double band at about 180 kD, and absence or low presence of a heavy chain band at about 80kD.

The expression levels and *in vitro* functionality of different single chain FVIII variants were evaluated in comparison to the double chain FVIII variant, AC-6rs-REF, having the identical amino acid sequence as ReFacto AF® and used as baseline molecule during the hemophilia A therapeutic development. Naturally, this double-chain FVIII should be expected to deliver the best functional results.

As described above, CAP-T cells were transiently transfected in duplicate with respective plasmid DNA encoding for either AC-6rs-REF or the different single chain molecules. After four days of cultivation, cells were centrifuged and cell culture supernatants were directly used for determining (I) the chromogenic FVIII activity, (II) the FVIII antigen corresponding to the total FVIII protein amount, and (III) the FVIII clotting activity induced via Actin FSL.

In a comparison of the constructs V1-V4 with the FVIII double-chain molecule AC-6rs (SEQ ID NO: 29), the chromogenic activity and specific chromogenic activity, demonstrating the ratio of chromogenic activity and FVIII antigen, of the expressed single chain constructs were lower than those of the double chain molecule. In more detail, the chromogenic activity and specific chromogenic activity of V3 and V4 were lower than that of V1 and V2. In turn, the chromogenic activity and specific chromogenic activity of the expressed constructs V1 and V2 was still lower than that of the double-chain molecule (data not shown).

In a comparison of V1, V2, V0, V5, V6, V7 and the double chain FVIII (AC-6rs-REF), as demonstrated in Fig. 3A, the double chain FVIII control reached the highest chromogenic FVIII activity levels of approx. 1 U/ml (all determined in supernatants from transfected cells). Single chain variants AC_SC-V0, -V5 and -V6 demonstrated chromogenic activities of approx. 0.7 U/ml while AC_SC-V1, -V2 and -V7 exhibited approx. 0.4 to 0.5 U/ml.

FVIII protein amounts were also found to be highest in the double chain AC-6rs-REF control with approx. 2.4 U/ml (Fig. 3C). Single chain variants were expressed in the range of 1.4 U/ml for AC_SC-V2 up to 2.0 U/ml for AC_SC-V5. Compared to the two stage chromogenic FVIII assay, the one stage clotting assay generally resulted in lower activity values (Fig. 3B). AC_SC-V0, AC-6rs-REF, AC_SC-V5, and AC_SC-V6 revealed approx. 0.3 U/ml while AC_SC-V7 demonstrated clotting activities of about 0.2 U/ml and AC_SC-V1 and -V2 demonstrated very low clotting activities.

Specific chromogenic activities represent the ratio of chromogenic FVIII activity and FVIII Antigen levels, i.e., they represent the decisive measure of activity. From the patient's view, a high specific activity is desirable, because it means that one can achieve better treatment with less material. In this context, the specific activity was calculated as chromogenic FVIII activity to FVIII antigen ratio displayed in %. As shown in Fig. 3D, the highest specific activity was observed for AC_SC-V0 (44%). The double chain AC-6rs-REF control reached a specific activity of 40%, followed by activities of AC_SC-V5,-V6 and -V7. The specific clotting activity was calculated as FVIII clotting activity to FVIII antigen ratio displayed in %. As shown in Fig. 3E, the highest specific activity was observed for AC_SC-V0 (22.8%) followed by -V6 (16.9%), -V5 (15.5%), -V7 (14.8%).The double chain AC-6rs-REF control reached a lower specific clotting activity of 13.9%, while the single chain constructs AC_SC-V1 and - V2 (prior art) reached only 6.7% and 9.6%, respectively.

In conclusion, the different single chain FVIII molecules AC_SC-V0, -V1, -V2, -V5, -V6, and - V7 were assessed for their *in vitro* functionality in comparison to the double chain FVIII, AC-6rs-REF. Therefore, all FVIII molecules were analyzed using a two stage chromogenic activity assay, a one stage clotting assay and a FVIII antigen ELISA detecting the total FVIII protein amount. It was observed that all FVIII variants were expressed, since FVIII antigen levels could be determined. In addition, all molecules were generally functional as activity values could be determined in both assays, chromogenic and clotting. However, lowest activities were determined for the single chain variants AC_SC-V1 and AC_SC-V2. While AC_SC-V7 performed moderate in those activity assays, AC_SC-V0, -V5, -V6, and -V7 performed best as single chain variants. The double chain AC-6rs-REF control overall presented the highest protein amount and chromogenic FVIII activity. The specific activities representing either the ratio of chromogenic activity to FVIII antigen or the ratio of clotting activity to FVIII antigen, both serving as indicator of protein functionality, were best for AC_SC-V0, AC_SC-V5, AC_SC-V6, and AC_SC-V7.

### 2. Stability and pharmacokinetic data

Prior to in vitro stability experiments and in vivo experiments, the FVIII single chain proteins were purified by standard purification procedures comprising cell removal and concentration of the supernatant, subsequent purification of the FVIII proteins via affinity chromatography, and re-buffering into FVIII formulation buffer.

Chromogenic and clotting activity of V0 and ReFacto AF® were analysed over 24 h and 14 days both in buffer and plasma lacking FVIII. V0 and ReFacto AF® were comparable with regard to stability (Fig. 4).

Pharmacokinetic data for V0 and the commercially available double chain FVIII ReFacto AF® were analysed in mice. Coagulation factors (200 IU (chromogenic activity)/kg body weight) were administered in 6 mL/kg body weight by a single intravenous tail vein injection into female haemophilia A mice (FVIII deficient mice, Jax No B6;129S-*F8^{tmlKaz}*/J, Charles River Laboratories, Sulzfeld, Germany). Blood sampling was performed 0.5, 4, 8, 12 and 20 h post treatment and citrate plasma was subsequently extracted by centrifugation. Plasma samples were analysed for the chromogenic FVIII activity and FVIII protein amount (FVIII antigen). Pharmacokinetic evaluation was performed by analysing the raw values and performing a non-compartmental analysis (NCA) using Phoenix WinNonlin 8.1 (Certara, USA).

During this study, ReFacto AF® attained a better-than-average half-life of approx. 7.4 h for antigen and 6.9 h for FVIII chromogenic activity. In comparison, V0 showed a half-life of 6.1 and 6.8 h for antigen and chromogenic activity, respectively. Maximal concentrations after intravenous administration were observed for both V0 and ReFacto AF® in the samples taken 0.5 h post injection with values of 2.16 IU/ml and 2.02 IU/ml, respectively. Thus, both variants show a comparable half-life both in relation to antigen and to chromogenic activity (Fig. 5) and comparable maximal concentrations after injection.

Thus, translating the in vitro findings to the animal model, the inventors were able to confirm the advantageous properties of the FVIII variants according to the invention also in the in vivo situation.

## Claims

1. A recombinant Factor VIII protein comprising, in a single chain, a heavy chain portion comprising an A1 and an A2 domain and a light chain portion comprising an A3, C1 and C2 domain of Factor VIII, wherein,
a) in said recombinant Factor VIII protein, 894 amino acids corresponding to consecutive amino acids between F761 and P1659 of wild type Factor VIII as defined in SEQ ID NO: 1 are deleted, leading to a first deletion;
b) said recombinant Factor VIII protein comprises, spanning the site of the first deletion, a processing sequence comprising SEQ ID NO: 2 or a sequence having at most one amino acid substitution in SEQ ID NO: 2, wherein said processing sequence comprises a first thrombin cleavage site;
c) in said recombinant Factor VIII protein, at least the amino acids corresponding to amino acids R1664 to R1667 of wild type Factor VIII are deleted, leading to a second deletion; and
d) said recombinant Factor VIII protein comprises, C-terminal to the second deletion and N-terminal of the A3 domain, a second thrombin cleavage site.

2. A recombinant Factor VIII protein comprising, in a single chain, a heavy chain portion comprising an A1 and an A2 domain and a light chain portion comprising an A3, C1 and C2 domain of Factor VIII, wherein,
a) said recombinant Factor VIII protein comprises a processing sequence comprising SEQ ID NO: 2 or a sequence having at most one amino acid substitution in SEQ ID NO: 2, wherein said processing sequence comprises a first thrombin cleavage site;
b) optionally, directly C-terminal to said processing sequence, said Factor VIII protein comprises a heterologous sequence;
c) directly C-terminal to said processing sequence, or, if present, directly C-terminal to said heterologous sequence, said Factor VIII protein comprises a merging sequence having at least 90% sequence identity to a sequence selected from the group consisting of SEQ ID NO: 9, SEQ ID NO: 10 and SEQ ID NO: 11; and
d) said recombinant Factor VIII protein comprises, C-terminal to SEQ ID NO: 9-11, a second thrombin cleavage site,
wherein said Factor VIII protein optionally is a Factor VIII protein of claim 1.

3. The recombinant Factor VIII protein of any one of the preceding claims, wherein the processing sequence is SEQ ID NO: 4 or a sequence having at most one amino acid substitution in said sequence, wherein, optionally, the F, the S C-terminal to the F, the Q or the N are substituted.

4. The recombinant Factor VIII protein of any one of the preceding claims, wherein the processing sequence is selected from the group consisting of SEQ ID NO: 4, 6, 7 or 8.

5. The recombinant Factor VIII protein of any one of the preceding claims, wherein the processing sequence is SEQ ID NO: 4.

6. The recombinant Factor VIII protein of any one of the preceding claims, wherein the amino acids corresponding to amino acids K1663 to L1674 of wild type Factor VIII are deleted, leading to a second deletion.

7. The recombinant Factor VIII protein of any one of the preceding claims, not comprising a furin cleavage recognition site, and not comprising a sequence having more than 75% sequence identity to the furin cleavage recognition site RHQR between the processing sequence and the merging sequence,
preferably, not comprising a sequence having more than 40% sequence identity to SEQ ID NO: 15.

8. The recombinant Factor VIII protein of any one of the preceding claims, comprising a processing sequence and, C-terminal to said processing sequence, a merging sequence, wherein said sequences are selected from the group consisting of
a. the processing sequence of SEQ ID NO: 4 and a merging sequence of SEQ ID NO: 12,
b. the processing sequence of SEQ ID NO: 5 and a merging sequence of SEQ ID NO: 12,
c. the processing sequence of SEQ ID NO: 6 and a merging sequence of SEQ ID NO: 12,
d. the processing sequence of SEQ ID NO: 7 and a merging sequence of SEQ ID NO: 12,
e. the processing sequence of SEQ ID NO: 8 and a merging sequence of SEQ ID NO: 12,
f. the processing sequence of SEQ ID NO: 3 and a merging sequence of SEQ ID NO: 13,
g. the processing sequence of SEQ ID NO: 2 and a merging sequence of SEQ ID NO: 13,
h. the processing sequence of SEQ ID NO: 3 and a merging sequence of SEQ ID NO: 14,
wherein, optionally, said merging sequence is directly C-terminal to said processing sequence.

9. The recombinant Factor VIII protein of any one of the preceding claims, further comprising a third thrombin cleavage site between the A1 and A2 domain.

10. The recombinant Factor VIII protein of any one of the preceding claims that is a fusion protein with at least one heterologous fusion partner selected from the group consisting of an Fc region, an albumin-binding sequence, albumin, PAS polypeptides, HAP polypeptides, the C-terminal peptide of the beta subunit of chorionic gonadotropin, albumin-binding small molecules, polyethylenglycol, hydroxyethyl starch,
wherein, optionally, said heterologous fusion partner is inserted directly C-terminal to said processing sequence.

11. A nucleic acid encoding a recombinant Factor VIII protein of any one of the preceding claims, wherein said polynucleotide optionally is an expression vector suitable for expression of said recombinant Factor VIII protein in a mammalian cell selected from the group comprising a human cell.

12. A host cell comprising a nucleic acid of claim 11, wherein preferably the host cell is a mammalian cell comprising an expression vector suitable for expression of said recombinant Factor VIII protein in said cell.

13. A method for preparing a Factor VIII protein, comprising culturing the host cell of claim 12 under conditions suitable for expression of the Factor VIII protein and isolating the Factor VIII protein, wherein the method optionally comprises formulating the Factor VIII protein as a pharmaceutical composition.

14. A pharmaceutical composition comprising the recombinant Factor VIII protein of any of claims 1-10, the nucleic acid of claim 11, or the host cell of claim 12.

15. The pharmaceutical composition of claim 14 for use in treatment of hemophilia A,
wherein, optionally, the treatment is immune tolerance induction.


SEQUENCE LISTING

<110> Biotest AG

<120> Single chain Factor VIII molecule

<130> 11995 P 6689 EP

<160> 38

<170> BiSSAP 1.3.6

<210> 1
<211> 2351
<212> PRT
<213> Homo sapiens

<220>
<221> SIGNAL
<222> 1..19
<223> signal

<220>
<223> wt human FVIII

<400> 1
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Ser Arg His Pro
        755                 760                 765
Ser Thr Arg Gln Lys Gln Phe Asn Ala Thr Thr Ile Pro Glu Asn Asp
    770                 775                 780
Ile Glu Lys Thr Asp Pro Trp Phe Ala His Arg Thr Pro Met Pro Lys

785                 790                 795                 800
Ile Gln Asn Val Ser Ser Ser Asp Leu Leu Met Leu Leu Arg Gln Ser
                805                 810                 815
Pro Thr Pro His Gly Leu Ser Leu Ser Asp Leu Gln Glu Ala Lys Tyr
            820                 825                 830
Glu Thr Phe Ser Asp Asp Pro Ser Pro Gly Ala Ile Asp Ser Asn Asn
        835                 840                 845
Ser Leu Ser Glu Met Thr His Phe Arg Pro Gln Leu His His Ser Gly
    850                 855                 860
Asp Met Val Phe Thr Pro Glu Ser Gly Leu Gln Leu Arg Leu Asn Glu
865                 870                 875                 880
Lys Leu Gly Thr Thr Ala Ala Thr Glu Leu Lys Lys Leu Asp Phe Lys
                885                 890                 895
Val Ser Ser Thr Ser Asn Asn Leu Ile Ser Thr Ile Pro Ser Asp Asn
            900                 905                 910
Leu Ala Ala Gly Thr Asp Asn Thr Ser Ser Leu Gly Pro Pro Ser Met
        915                 920                 925
Pro Val His Tyr Asp Ser Gln Leu Asp Thr Thr Leu Phe Gly Lys Lys
    930                 935                 940
Ser Ser Pro Leu Thr Glu Ser Gly Gly Pro Leu Ser Leu Ser Glu Glu
945                 950                 955                 960
Asn Asn Asp Ser Lys Leu Leu Glu Ser Gly Leu Met Asn Ser Gln Glu
                965                 970                 975
Ser Ser Trp Gly Lys Asn Val Ser Ser Thr Glu Ser Gly Arg Leu Phe
            980                 985                 990
Lys Gly Lys Arg Ala His Gly Pro Ala Leu Leu Thr Lys Asp Asn Ala
        995                 1000                1005
Leu Phe Lys Val Ser Ile Ser Leu Leu Lys Thr Asn Lys Thr Ser Asn
    1010                1015                1020
Asn Ser Ala Thr Asn Arg Lys Thr His Ile Asp Gly Pro Ser Leu Leu
1025                1030                1035                1040
Ile Glu Asn Ser Pro Ser Val Trp Gln Asn Ile Leu Glu Ser Asp Thr
                1045                1050                1055
Glu Phe Lys Lys Val Thr Pro Leu Ile His Asp Arg Met Leu Met Asp
            1060                1065                1070
Lys Asn Ala Thr Ala Leu Arg Leu Asn His Met Ser Asn Lys Thr Thr
        1075                1080                1085
Ser Ser Lys Asn Met Glu Met Val Gln Gln Lys Lys Glu Gly Pro Ile
    1090                1095                1100
Pro Pro Asp Ala Gln Asn Pro Asp Met Ser Phe Phe Lys Met Leu Phe
1105                1110                1115                1120
Leu Pro Glu Ser Ala Arg Trp Ile Gln Arg Thr His Gly Lys Asn Ser
                1125                1130                1135
Leu Asn Ser Gly Gln Gly Pro Ser Pro Lys Gln Leu Val Ser Leu Gly
            1140                1145                1150
Pro Glu Lys Ser Val Glu Gly Gln Asn Phe Leu Ser Glu Lys Asn Lys
        1155                1160                1165
Val Val Val Gly Lys Gly Glu Phe Thr Lys Asp Val Gly Leu Lys Glu
    1170                1175                1180
Met Val Phe Pro Ser Ser Arg Asn Leu Phe Leu Thr Asn Leu Asp Asn
1185                1190                1195                1200
Leu His Glu Asn Asn Thr His Asn Gln Glu Lys Lys Ile Gln Glu Glu
                1205                1210                1215
Ile Glu Lys Lys Glu Thr Leu Ile Gln Glu Asn Val Val Leu Pro Gln
            1220                1225                1230
Ile His Thr Val Thr Gly Thr Lys Asn Phe Met Lys Asn Leu Phe Leu
        1235                1240                1245
Leu Ser Thr Arg Gln Asn Val Glu Gly Ser Tyr Asp Gly Ala Tyr Ala
    1250                1255                1260
Pro Val Leu Gln Asp Phe Arg Ser Leu Asn Asp Ser Thr Asn Arg Thr
1265                1270                1275                1280
Lys Lys His Thr Ala His Phe Ser Lys Lys Gly Glu Glu Glu Asn Leu
                1285                1290                1295

Glu Gly Leu Gly Asn Gln Thr Lys Gln Ile Val Glu Lys Tyr Ala Cys
            1300                1305                1310
Thr Thr Arg Ile Ser Pro Asn Thr Ser Gln Gln Asn Phe Val Thr Gln
        1315                1320                1325
Arg Ser Lys Arg Ala Leu Lys Gln Phe Arg Leu Pro Leu Glu Glu Thr
    1330                1335                1340
Glu Leu Glu Lys Arg Ile Ile Val Asp Asp Thr Ser Thr Gln Trp Ser
1345                1350                1355                1360
Lys Asn Met Lys His Leu Thr Pro Ser Thr Leu Thr Gln Ile Asp Tyr
                1365                1370                1375
Asn Glu Lys Glu Lys Gly Ala Ile Thr Gln Ser Pro Leu Ser Asp Cys
            1380                1385                1390
Leu Thr Arg Ser His Ser Ile Pro Gln Ala Asn Arg Ser Pro Leu Pro
        1395                1400                1405
Ile Ala Lys Val Ser Ser Phe Pro Ser Ile Arg Pro Ile Tyr Leu Thr
    1410                1415                1420
Arg Val Leu Phe Gln Asp Asn Ser Ser His Leu Pro Ala Ala Ser Tyr
1425                1430                1435                1440
Arg Lys Lys Asp Ser Gly Val Gln Glu Ser Ser His Phe Leu Gln Gly
                1445                1450                1455
Ala Lys Lys Asn Asn Leu Ser Leu Ala Ile Leu Thr Leu Glu Met Thr
            1460                1465                1470
Gly Asp Gln Arg Glu Val Gly Ser Leu Gly Thr Ser Ala Thr Asn Ser
        1475                1480                1485
Val Thr Tyr Lys Lys Val Glu Asn Thr Val Leu Pro Lys Pro Asp Leu
    1490                1495                1500
Pro Lys Thr Ser Gly Lys Val Glu Leu Leu Pro Lys Val His Ile Tyr
1505                1510                1515                1520
Gln Lys Asp Leu Phe Pro Thr Glu Thr Ser Asn Gly Ser Pro Gly His
                1525                1530                1535
Leu Asp Leu Val Glu Gly Ser Leu Leu Gln Gly Thr Glu Gly Ala Ile
            1540                1545                1550
Lys Trp Asn Glu Ala Asn Arg Pro Gly Lys Val Pro Phe Leu Arg Val
        1555                1560                1565
Ala Thr Glu Ser Ser Ala Lys Thr Pro Ser Lys Leu Leu Asp Pro Leu
    1570                1575                1580
Ala Trp Asp Asn His Tyr Gly Thr Gln Ile Pro Lys Glu Glu Trp Lys
1585                1590                1595                1600
Ser Gln Glu Lys Ser Pro Glu Lys Thr Ala Phe Lys Lys Lys Asp Thr
                1605                1610                1615
Ile Leu Ser Leu Asn Ala Cys Glu Ser Asn His Ala Ile Ala Ala Ile
            1620                1625                1630
Asn Glu Gly Gln Asn Lys Pro Glu Ile Glu Val Thr Trp Ala Lys Gln
        1635                1640                1645
Gly Arg Thr Glu Arg Leu Cys Ser Gln Asn Pro Pro Val Leu Lys Arg
    1650                1655                1660
His Gln Arg Glu Ile Thr Arg Thr Thr Leu Gln Ser Asp Gln Glu Glu
1665                1670                1675                1680
Ile Asp Tyr Asp Asp Thr Ile Ser Val Glu Met Lys Lys Glu Asp Phe
                1685                1690                1695
Asp Ile Tyr Asp Glu Asp Glu Asn Gln Ser Pro Arg Ser Phe Gln Lys
            1700                1705                1710
Lys Thr Arg His Tyr Phe Ile Ala Ala Val Glu Arg Leu Trp Asp Tyr
        1715                1720                1725
Gly Met Ser Ser Ser Pro His Val Leu Arg Asn Arg Ala Gln Ser Gly
    1730                1735                1740
Ser Val Pro Gln Phe Lys Lys Val Val Phe Gln Glu Phe Thr Asp Gly
1745                1750                1755                1760
Ser Phe Thr Gln Pro Leu Tyr Arg Gly Glu Leu Asn Glu His Leu Gly
                1765                1770                1775
Leu Leu Gly Pro Tyr Ile Arg Ala Glu Val Glu Asp Asn Ile Met Val
            1780                1785                1790
Thr Phe Arg Asn Gln Ala Ser Arg Pro Tyr Ser Phe Tyr Ser Ser Leu

1795                1800                1805
Ile Ser Tyr Glu Glu Asp Gln Arg Gln Gly Ala Glu Pro Arg Lys Asn
    1810                1815                1820
Phe Val Lys Pro Asn Glu Thr Lys Thr Tyr Phe Trp Lys Val Gln His
1825                1830                1835                1840
His Met Ala Pro Thr Lys Asp Glu Phe Asp Cys Lys Ala Trp Ala Tyr
                1845                1850                1855
Phe Ser Asp Val Asp Leu Glu Lys Asp Val His Ser Gly Leu Ile Gly
            1860                1865                1870
Pro Leu Leu Val Cys His Thr Asn Thr Leu Asn Pro Ala His Gly Arg
        1875                1880                1885
Gln Val Thr Val Gln Glu Phe Ala Leu Phe Phe Thr Ile Phe Asp Glu
    1890                1895                1900
Thr Lys Ser Trp Tyr Phe Thr Glu Asn Met Glu Arg Asn Cys Arg Ala
1905                1910                1915                1920
Pro Cys Asn Ile Gln Met Glu Asp Pro Thr Phe Lys Glu Asn Tyr Arg
                1925                1930                1935
Phe His Ala Ile Asn Gly Tyr Ile Met Asp Thr Leu Pro Gly Leu Val
            1940                1945                1950
Met Ala Gln Asp Gln Arg Ile Arg Trp Tyr Leu Leu Ser Met Gly Ser
        1955                1960                1965
Asn Glu Asn Ile His Ser Ile His Phe Ser Gly His Val Phe Thr Val
    1970                1975                1980
Arg Lys Lys Glu Glu Tyr Lys Met Ala Leu Tyr Asn Leu Tyr Pro Gly
1985                1990                1995                2000
Val Phe Glu Thr Val Glu Met Leu Pro Ser Lys Ala Gly Ile Trp Arg
                2005                2010                2015
Val Glu Cys Leu Ile Gly Glu His Leu His Ala Gly Met Ser Thr Leu
            2020                2025                2030
Phe Leu Val Tyr Ser Asn Lys Cys Gln Thr Pro Leu Gly Met Ala Ser
        2035                2040                2045
Gly His Ile Arg Asp Phe Gln Ile Thr Ala Ser Gly Gln Tyr Gly Gln
    2050                2055                2060
Trp Ala Pro Lys Leu Ala Arg Leu His Tyr Ser Gly Ser Ile Asn Ala
2065                2070                2075                2080
Trp Ser Thr Lys Glu Pro Phe Ser Trp Ile Lys Val Asp Leu Leu Ala
                2085                2090                2095
Pro Met Ile Ile His Gly Ile Lys Thr Gln Gly Ala Arg Gln Lys Phe
            2100                2105                2110
Ser Ser Leu Tyr Ile Ser Gln Phe Ile Ile Met Tyr Ser Leu Asp Gly
        2115                2120                2125
Lys Lys Trp Gln Thr Tyr Arg Gly Asn Ser Thr Gly Thr Leu Met Val
    2130                2135                2140
Phe Phe Gly Asn Val Asp Ser Ser Gly Ile Lys His Asn Ile Phe Asn
2145                2150                2155                2160
Pro Pro Ile Ile Ala Arg Tyr Ile Arg Leu His Pro Thr His Tyr Ser
                2165                2170                2175
Ile Arg Ser Thr Leu Arg Met Glu Leu Met Gly Cys Asp Leu Asn Ser
            2180                2185                2190
Cys Ser Met Pro Leu Gly Met Glu Ser Lys Ala Ile Ser Asp Ala Gln
        2195                2200                2205
Ile Thr Ala Ser Ser Tyr Phe Thr Asn Met Phe Ala Thr Trp Ser Pro
    2210                2215                2220
Ser Lys Ala Arg Leu His Leu Gln Gly Arg Ser Asn Ala Trp Arg Pro
2225                2230                2235                2240
Gln Val Asn Asn Pro Lys Glu Trp Leu Gln Val Asp Phe Gln Lys Thr
                2245                2250                2255
Met Lys Val Thr Gly Val Thr Thr Gln Gly Val Lys Ser Leu Leu Thr
            2260                2265                2270
Ser Met Tyr Val Lys Glu Phe Leu Ile Ser Ser Ser Gln Asp Gly His
        2275                2280                2285
Gln Trp Thr Leu Phe Phe Gln Asn Gly Lys Val Lys Val Phe Gln Gly
    2290                2295                2300

Asn Gln Asp Ser Phe Thr Pro Val Val Asn Ser Leu Asp Pro Pro Leu
2305                2310                2315                2320
Leu Thr Arg Tyr Leu Arg Ile His Pro Gln Ser Trp Val His Gln Ile
                2325                2330                2335
Ala Leu Arg Met Glu Val Leu Gly Cys Glu Ala Gln Asp Leu Tyr
            2340                2345                2350

<210> 2
<211> 9
<212> PRT
<213> Artificial Sequence

<220>
<223> minimal processing sequence

<400> 2
Pro Arg Ser Phe Ser Gln Asn Pro Pro
1               5

<210> 3
<211> 10
<212> PRT
<213> Artificial Sequence

<220>
<223> Processing sequence

<400> 3
Pro Arg Ser Phe Ser Gln Asn Pro Pro Val
1               5                   10

<210> 4
<211> 11
<212> PRT
<213> Artificial Sequence

<220>
<223> Processing sequence

<400> 4
Pro Arg Ser Phe Ser Gln Asn Pro Pro Val Leu
1               5                   10

<210> 5
<211> 11
<212> PRT
<213> Artificial Sequence

<220>
<223> Processing sequence

<220>
<221> VARIANT
<222> 4

<400> 5
Pro Arg Ser Xaa Ser Gln Asn Pro Pro Val Leu
1               5                   10

<210> 6
<211> 11
<212> PRT
<213> Artificial Sequence

<220>
<223> Processing sequence

<220>
<221> VARIANT
<222> 5

<400> 6
Pro Arg Ser Phe Xaa Gln Asn Pro Pro Val Leu
1               5                   10

<210> 7
<211> 11
<212> PRT
<213> Artificial Sequence

<220>
<223> Processing sequence

<220>
<221> VARIANT
<222> 6

<400> 7
Pro Arg Ser Phe Ser Xaa Asn Pro Pro Val Leu
1               5                   10

<210> 8
<211> 11
<212> PRT
<213> Artificial Sequence

<220>
<223> Processing sequence

<220>
<221> VARIANT
<222> 7

<400> 8
Pro Arg Ser Phe Ser Gln Xaa Pro Pro Val Leu
1               5                   10

<210> 9
<211> 10
<212> PRT
<213> Artificial Sequence

<220>
<223> Merging sequence, e.g., in V0

<400> 9
Gln Ser Asp Gln Glu Glu Ile Asp Tyr Asp
1               5                   10

<210> 10
<211> 7
<212> PRT
<213> Artificial Sequence

<220>
<223> merging sequence, e.g., in V5 + V6

<400> 10
Ile Asp Tyr Asp Asp Thr Ile
1               5

<210> 11
<211> 8
<212> PRT
<213> Artificial Sequence

<220>
<223> merging sequence, e.g., in V7

<400> 11
Glu Met Lys Lys Glu Asp Phe Asp
1               5

<210> 12
<211> 34
<212> PRT
<213> Artificial Sequence

<220>
<223> merging sequence, e.g., in V0

<400> 12
Gln Ser Asp Gln Glu Glu Ile Asp Tyr Asp Asp Thr Ile Ser Val Glu
1               5                   10                  15
Met Lys Lys Glu Asp Phe Asp Ile Tyr Asp Glu Asp Glu Asn Gln Ser
            20                  25                  30
Pro Arg

<210> 13
<211> 28
<212> PRT
<213> Artificial Sequence

<220>
<223> merging sequence, e.g., in V6

<400> 13
Ile Asp Tyr Asp Asp Thr Ile Ser Val Glu Met Lys Lys Glu Asp Phe
1               5                   10                  15
Asp Ile Tyr Asp Glu Asp Glu Asn Gln Ser Pro Arg
            20                  25

<210> 14
<211> 19
<212> PRT
<213> Artificial Sequence

<220>
<223> merging sequence, e.g., in V7

<400> 14
Glu Met Lys Lys Glu Asp Phe Asp Ile Tyr Asp Glu Asp Glu Asn Gln
1               5                   10                  15
Ser Pro Arg


<210> 15
<211> 11
<212> PRT
<213> Homo sapiens


<220>
<223> FVIII sequence comprising furin cleavage recognition site deleted
      in proteins of the invention

<400> 15
Lys Arg His Gln Arg Glu Ile Thr Arg Thr Thr
1               5                   10

<210> 16
<211> 1445
<212> PRT
<213> Artificial Sequence


<220>
<221> SIGNAL
<222> 1..19

<220>
<223> V0 aa incl. signal sequence

<400> 16
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro

690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Gln Ser Asp Gln Glu Glu Ile Asp Tyr Asp Asp Thr Ile Ser Val Glu
    770                 775                 780
Met Lys Lys Glu Asp Phe Asp Ile Tyr Asp Glu Asp Glu Asn Gln Ser
785                 790                 795                 800
Pro Arg Ser Phe Gln Lys Lys Thr Arg His Tyr Phe Ile Ala Ala Val
                805                 810                 815
Glu Arg Leu Trp Asp Tyr Gly Met Ser Ser Ser Pro His Val Leu Arg
            820                 825                 830
Asn Arg Ala Gln Ser Gly Ser Val Pro Gln Phe Lys Lys Val Val Phe
        835                 840                 845
Gln Glu Phe Thr Asp Gly Ser Phe Thr Gln Pro Leu Tyr Arg Gly Glu
    850                 855                 860
Leu Asn Glu His Leu Gly Leu Leu Gly Pro Tyr Ile Arg Ala Glu Val
865                 870                 875                 880
Glu Asp Asn Ile Met Val Thr Phe Arg Asn Gln Ala Ser Arg Pro Tyr
                885                 890                 895
Ser Phe Tyr Ser Ser Leu Ile Ser Tyr Glu Glu Asp Gln Arg Gln Gly
            900                 905                 910
Ala Glu Pro Arg Lys Asn Phe Val Lys Pro Asn Glu Thr Lys Thr Tyr
        915                 920                 925
Phe Trp Lys Val Gln His His Met Ala Pro Thr Lys Asp Glu Phe Asp
    930                 935                 940
Cys Lys Ala Trp Ala Tyr Phe Ser Asp Val Asp Leu Glu Lys Asp Val
945                 950                 955                 960
His Ser Gly Leu Ile Gly Pro Leu Leu Val Cys His Thr Asn Thr Leu
                965                 970                 975
Asn Pro Ala His Gly Arg Gln Val Thr Val Gln Glu Phe Ala Leu Phe
            980                 985                 990
Phe Thr Ile Phe Asp Glu Thr Lys Ser Trp Tyr Phe Thr Glu Asn Met
        995                 1000                1005
Glu Arg Asn Cys Arg Ala Pro Cys Asn Ile Gln Met Glu Asp Pro Thr
    1010                1015                1020
Phe Lys Glu Asn Tyr Arg Phe His Ala Ile Asn Gly Tyr Ile Met Asp
1025                1030                1035                1040
Thr Leu Pro Gly Leu Val Met Ala Gln Asp Gln Arg Ile Arg Trp Tyr
                1045                1050                1055
Leu Leu Ser Met Gly Ser Asn Glu Asn Ile His Ser Ile His Phe Ser
            1060                1065                1070
Gly His Val Phe Thr Val Arg Lys Lys Glu Glu Tyr Lys Met Ala Leu
        1075                1080                1085
Tyr Asn Leu Tyr Pro Gly Val Phe Glu Thr Val Glu Met Leu Pro Ser
    1090                1095                1100
Lys Ala Gly Ile Trp Arg Val Glu Cys Leu Ile Gly Glu His Leu His
1105                1110                1115                1120
Ala Gly Met Ser Thr Leu Phe Leu Val Tyr Ser Asn Lys Cys Gln Thr
                1125                1130                1135
Pro Leu Gly Met Ala Ser Gly His Ile Arg Asp Phe Gln Ile Thr Ala
            1140                1145                1150
Ser Gly Gln Tyr Gly Gln Trp Ala Pro Lys Leu Ala Arg Leu His Tyr
        1155                1160                1165
Ser Gly Ser Ile Asn Ala Trp Ser Thr Lys Glu Pro Phe Ser Trp Ile
    1170                1175                1180
Lys Val Asp Leu Leu Ala Pro Met Ile Ile His Gly Ile Lys Thr Gln
1185                1190                1195                1200

Gly Ala Arg Gln Lys Phe Ser Ser Leu Tyr Ile Ser Gln Phe Ile Ile
                1205                1210                1215
Met Tyr Ser Leu Asp Gly Lys Lys Trp Gln Thr Tyr Arg Gly Asn Ser
            1220                1225                1230
Thr Gly Thr Leu Met Val Phe Phe Gly Asn Val Asp Ser Ser Gly Ile
        1235                1240                1245
Lys His Asn Ile Phe Asn Pro Pro Ile Ile Ala Arg Tyr Ile Arg Leu
    1250                1255                1260
His Pro Thr His Tyr Ser Ile Arg Ser Thr Leu Arg Met Glu Leu Met
1265                1270                1275                1280
Gly Cys Asp Leu Asn Ser Cys Ser Met Pro Leu Gly Met Glu Ser Lys
                1285                1290                1295
Ala Ile Ser Asp Ala Gln Ile Thr Ala Ser Ser Tyr Phe Thr Asn Met
            1300                1305                1310
Phe Ala Thr Trp Ser Pro Ser Lys Ala Arg Leu His Leu Gln Gly Arg
        1315                1320                1325
Ser Asn Ala Trp Arg Pro Gln Val Asn Asn Pro Lys Glu Trp Leu Gln
    1330                1335                1340
Val Asp Phe Gln Lys Thr Met Lys Val Thr Gly Val Thr Thr Gln Gly
1345                1350                1355                1360
Val Lys Ser Leu Leu Thr Ser Met Tyr Val Lys Glu Phe Leu Ile Ser
                1365                1370                1375
Ser Ser Gln Asp Gly His Gln Trp Thr Leu Phe Phe Gln Asn Gly Lys
            1380                1385                1390
Val Lys Val Phe Gln Gly Asn Gln Asp Ser Phe Thr Pro Val Val Asn
        1395                1400                1405
Ser Leu Asp Pro Pro Leu Leu Thr Arg Tyr Leu Arg Ile His Pro Gln
    1410                1415                1420
Ser Trp Val His Gln Ile Ala Leu Arg Met Glu Val Leu Gly Cys Glu
1425                1430                1435                1440
Ala Gln Asp Leu Tyr
                1445

<210> 17
<211> 1423
<212> PRT
<213> Artificial Sequence

<220>
<221> SIGNAL
<222> 1..19

<220>
<223> V1 aa incl. signal sequence

<400> 17
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val

625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Val Glu Met Lys Lys Glu Asp Phe
        755                 760                 765
Asp Ile Tyr Asp Glu Asp Glu Asn Gln Ser Pro Arg Ser Phe Gln Lys
    770                 775                 780
Lys Thr Arg His Tyr Phe Ile Ala Ala Val Glu Arg Leu Trp Asp Tyr
785                 790                 795                 800
Gly Met Ser Ser Ser Pro His Val Leu Arg Asn Arg Ala Gln Ser Gly
                805                 810                 815
Ser Val Pro Gln Phe Lys Lys Val Val Phe Gln Glu Phe Thr Asp Gly
            820                 825                 830
Ser Phe Thr Gln Pro Leu Tyr Arg Gly Glu Leu Asn Glu His Leu Gly
        835                 840                 845
Leu Leu Gly Pro Tyr Ile Arg Ala Glu Val Glu Asp Asn Ile Met Val
    850                 855                 860
Thr Phe Arg Asn Gln Ala Ser Arg Pro Tyr Ser Phe Tyr Ser Ser Leu
865                 870                 875                 880
Ile Ser Tyr Glu Glu Asp Gln Arg Gln Gly Ala Glu Pro Arg Lys Asn
                885                 890                 895
Phe Val Lys Pro Asn Glu Thr Lys Thr Tyr Phe Trp Lys Val Gln His
            900                 905                 910
His Met Ala Pro Thr Lys Asp Glu Phe Asp Cys Lys Ala Trp Ala Tyr
        915                 920                 925
Phe Ser Asp Val Asp Leu Glu Lys Asp Val His Ser Gly Leu Ile Gly
    930                 935                 940
Pro Leu Leu Val Cys His Thr Asn Thr Leu Asn Pro Ala His Gly Arg
945                 950                 955                 960
Gln Val Thr Val Gln Glu Phe Ala Leu Phe Phe Thr Ile Phe Asp Glu
                965                 970                 975
Thr Lys Ser Trp Tyr Phe Thr Glu Asn Met Glu Arg Asn Cys Arg Ala
            980                 985                 990
Pro Cys Asn Ile Gln Met Glu Asp Pro Thr Phe Lys Glu Asn Tyr Arg
        995                 1000                1005
Phe His Ala Ile Asn Gly Tyr Ile Met Asp Thr Leu Pro Gly Leu Val
    1010                1015                1020
Met Ala Gln Asp Gln Arg Ile Arg Trp Tyr Leu Leu Ser Met Gly Ser
1025                1030                1035                1040
Asn Glu Asn Ile His Ser Ile His Phe Ser Gly His Val Phe Thr Val
                1045                1050                1055
Arg Lys Lys Glu Glu Tyr Lys Met Ala Leu Tyr Asn Leu Tyr Pro Gly
            1060                1065                1070
Val Phe Glu Thr Val Glu Met Leu Pro Ser Lys Ala Gly Ile Trp Arg
        1075                1080                1085
Val Glu Cys Leu Ile Gly Glu His Leu His Ala Gly Met Ser Thr Leu
    1090                1095                1100
Phe Leu Val Tyr Ser Asn Lys Cys Gln Thr Pro Leu Gly Met Ala Ser
1105                1110                1115                1120
Gly His Ile Arg Asp Phe Gln Ile Thr Ala Ser Gly Gln Tyr Gly Gln
                1125                1130                1135

Trp Ala Pro Lys Leu Ala Arg Leu His Tyr Ser Gly Ser Ile Asn Ala
            1140                1145                1150
Trp Ser Thr Lys Glu Pro Phe Ser Trp Ile Lys Val Asp Leu Leu Ala
        1155                1160                1165
Pro Met Ile Ile His Gly Ile Lys Thr Gln Gly Ala Arg Gln Lys Phe
    1170                1175                1180
Ser Ser Leu Tyr Ile Ser Gln Phe Ile Ile Met Tyr Ser Leu Asp Gly
1185                1190                1195                1200
Lys Lys Trp Gln Thr Tyr Arg Gly Asn Ser Thr Gly Thr Leu Met Val
                1205                1210                1215
Phe Phe Gly Asn Val Asp Ser Ser Gly Ile Lys His Asn Ile Phe Asn
            1220                1225                1230
Pro Pro Ile Ile Ala Arg Tyr Ile Arg Leu His Pro Thr His Tyr Ser
        1235                1240                1245
Ile Arg Ser Thr Leu Arg Met Glu Leu Met Gly Cys Asp Leu Asn Ser
    1250                1255                1260
Cys Ser Met Pro Leu Gly Met Glu Ser Lys Ala Ile Ser Asp Ala Gln
1265                1270                1275                1280
Ile Thr Ala Ser Ser Tyr Phe Thr Asn Met Phe Ala Thr Trp Ser Pro
                1285                1290                1295
Ser Lys Ala Arg Leu His Leu Gln Gly Arg Ser Asn Ala Trp Arg Pro
            1300                1305                1310
Gln Val Asn Asn Pro Lys Glu Trp Leu Gln Val Asp Phe Gln Lys Thr
        1315                1320                1325
Met Lys Val Thr Gly Val Thr Thr Gln Gly Val Lys Ser Leu Leu Thr
    1330                1335                1340
Ser Met Tyr Val Lys Glu Phe Leu Ile Ser Ser Ser Gln Asp Gly His
1345                1350                1355                1360
Gln Trp Thr Leu Phe Phe Gln Asn Gly Lys Val Lys Val Phe Gln Gly
                1365                1370                1375
Asn Gln Asp Ser Phe Thr Pro Val Val Asn Ser Leu Asp Pro Pro Leu
            1380                1385                1390
Leu Thr Arg Tyr Leu Arg Ile His Pro Gln Ser Trp Val His Gln Ile
        1395                1400                1405
Ala Leu Arg Met Glu Val Leu Gly Cys Glu Ala Gln Asp Leu Tyr
    1410                1415                1420

<210> 18
<211> 1425
<212> PRT
<213> Artificial Sequence

<220>
<221> SIGNAL
<222> 1..19

<220>
<223> V2 aa incl. signal sequence

<400> 18
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
                100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
                180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
                260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
                340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
                420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
                500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
                580                 585                 590
Ile Leu Phe Ser Val Phe Asp Glu Asn Arg Ser Trp Tyr Leu Thr Glu

595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Val Ala Ser Val Glu Met Lys Lys Glu
        755                 760                 765
Asp Phe Asp Ile Tyr Asp Glu Asp Glu Asn Gln Ser Pro Arg Ser Phe
    770                 775                 780
Gln Lys Lys Thr Arg His Tyr Phe Ile Ala Ala Val Glu Arg Leu Trp
785                 790                 795                 800
Asp Tyr Gly Met Ser Ser Ser Pro His Val Leu Arg Asn Arg Ala Gln
                805                 810                 815
Ser Gly Ser Val Pro Gln Phe Lys Lys Val Val Phe Gln Glu Phe Thr
            820                 825                 830
Asp Gly Ser Phe Thr Gln Pro Leu Tyr Arg Gly Glu Leu Asn Glu His
        835                 840                 845
Leu Gly Leu Leu Gly Pro Tyr Ile Arg Ala Glu Val Glu Asp Asn Ile
    850                 855                 860
Met Val Thr Phe Arg Asn Gln Ala Ser Arg Pro Tyr Ser Phe Tyr Ser
865                 870                 875                 880
Ser Leu Ile Ser Tyr Glu Glu Asp Gln Arg Gln Gly Ala Glu Pro Arg
                885                 890                 895
Lys Asn Phe Val Lys Pro Asn Glu Thr Lys Thr Tyr Phe Trp Lys Val
            900                 905                 910
Gln His His Met Ala Pro Thr Lys Asp Glu Phe Asp Cys Lys Ala Trp
        915                 920                 925
Ala Tyr Phe Ser Asp Val Asp Leu Glu Lys Asp Val His Ser Gly Leu
    930                 935                 940
Ile Gly Pro Leu Leu Val Cys His Thr Asn Thr Leu Asn Pro Ala His
945                 950                 955                 960
Gly Arg Gln Val Thr Val Gln Glu Phe Ala Leu Phe Phe Thr Ile Phe
                965                 970                 975
Asp Glu Thr Lys Ser Trp Tyr Phe Thr Glu Asn Met Glu Arg Asn Cys
            980                 985                 990
Arg Ala Pro Cys Asn Ile Gln Met Glu Asp Pro Thr Phe Lys Glu Asn
        995                 1000                1005
Tyr Arg Phe His Ala Ile Asn Gly Tyr Ile Met Asp Thr Leu Pro Gly
    1010                1015                1020
Leu Val Met Ala Gln Asp Gln Arg Ile Arg Trp Tyr Leu Leu Ser Met
1025                1030                1035                1040
Gly Ser Asn Glu Asn Ile His Ser Ile His Phe Ser Gly His Val Phe
                1045                1050                1055
Thr Val Arg Lys Lys Glu Glu Tyr Lys Met Ala Leu Tyr Asn Leu Tyr
            1060                1065                1070
Pro Gly Val Phe Glu Thr Val Glu Met Leu Pro Ser Lys Ala Gly Ile
        1075                1080                1085
Trp Arg Val Glu Cys Leu Ile Gly Glu His Leu His Ala Gly Met Ser
    1090                1095                1100

Thr Leu Phe Leu Val Tyr Ser Asn Lys Cys Gln Thr Pro Leu Gly Met
1105                1110                1115                1120
Ala Ser Gly His Ile Arg Asp Phe Gln Ile Thr Ala Ser Gly Gln Tyr
                1125                1130                1135
Gly Gln Trp Ala Pro Lys Leu Ala Arg Leu His Tyr Ser Gly Ser Ile
            1140                1145                1150
Asn Ala Trp Ser Thr Lys Glu Pro Phe Ser Trp Ile Lys Val Asp Leu
        1155                1160                1165
Leu Ala Pro Met Ile Ile His Gly Ile Lys Thr Gln Gly Ala Arg Gln
    1170                1175                1180
Lys Phe Ser Ser Leu Tyr Ile Ser Gln Phe Ile Ile Met Tyr Ser Leu
1185                1190                1195                1200
Asp Gly Lys Lys Trp Gln Thr Tyr Arg Gly Asn Ser Thr Gly Thr Leu
                1205                1210                1215
Met Val Phe Phe Gly Asn Val Asp Ser Ser Gly Ile Lys His Asn Ile
            1220                1225                1230
Phe Asn Pro Pro Ile Ile Ala Arg Tyr Ile Arg Leu His Pro Thr His
        1235                1240                1245
Tyr Ser Ile Arg Ser Thr Leu Arg Met Glu Leu Met Gly Cys Asp Leu
    1250                1255                1260
Asn Ser Cys Ser Met Pro Leu Gly Met Glu Ser Lys Ala Ile Ser Asp
1265                1270                1275                1280
Ala Gln Ile Thr Ala Ser Ser Tyr Phe Thr Asn Met Phe Ala Thr Trp
                1285                1290                1295
Ser Pro Ser Lys Ala Arg Leu His Leu Gln Gly Arg Ser Asn Ala Trp
            1300                1305                1310
Arg Pro Gln Val Asn Asn Pro Lys Glu Trp Leu Gln Val Asp Phe Gln
        1315                1320                1325
Lys Thr Met Lys Val Thr Gly Val Thr Thr Gln Gly Val Lys Ser Leu
    1330                1335                1340
Leu Thr Ser Met Tyr Val Lys Glu Phe Leu Ile Ser Ser Ser Gln Asp
1345                1350                1355                1360
Gly His Gln Trp Thr Leu Phe Phe Gln Asn Gly Lys Val Lys Val Phe
                1365                1370                1375
Gln Gly Asn Gln Asp Ser Phe Thr Pro Val Val Asn Ser Leu Asp Pro
            1380                1385                1390
Pro Leu Leu Thr Arg Tyr Leu Arg Ile His Pro Gln Ser Trp Val His
        1395                1400                1405
Gln Ile Ala Leu Arg Met Glu Val Leu Gly Cys Glu Ala Gln Asp Leu
    1410                1415                1420
Tyr
1425

<210> 19
<211> 1394
<212> PRT
<213> Artificial Sequence

<220>
<221> SIGNAL
<222> 1..19

<220>
<223> V3 aa incl. signal

<400> 19
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg

545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Ser Val Glu Met Lys Lys
                725                 730                 735
Glu Asp Phe Asp Ile Tyr Asp Glu Asp Glu Asn Gln Ser Pro Arg Ser
            740                 745                 750
Phe Gln Lys Lys Thr Arg His Tyr Phe Ile Ala Ala Val Glu Arg Leu
        755                 760                 765
Trp Asp Tyr Gly Met Ser Ser Ser Pro His Val Leu Arg Asn Arg Ala
    770                 775                 780
Gln Ser Gly Ser Val Pro Gln Phe Lys Lys Val Val Phe Gln Glu Phe
785                 790                 795                 800
Thr Asp Gly Ser Phe Thr Gln Pro Leu Tyr Arg Gly Glu Leu Asn Glu
                805                 810                 815
His Leu Gly Leu Leu Gly Pro Tyr Ile Arg Ala Glu Val Glu Asp Asn
            820                 825                 830
Ile Met Val Thr Phe Arg Asn Gln Ala Ser Arg Pro Tyr Ser Phe Tyr
        835                 840                 845
Ser Ser Leu Ile Ser Tyr Glu Glu Asp Gln Arg Gln Gly Ala Glu Pro
    850                 855                 860
Arg Lys Asn Phe Val Lys Pro Asn Glu Thr Lys Thr Tyr Phe Trp Lys
865                 870                 875                 880
Val Gln His His Met Ala Pro Thr Lys Asp Glu Phe Asp Cys Lys Ala
                885                 890                 895
Trp Ala Tyr Phe Ser Asp Val Asp Leu Glu Lys Asp Val His Ser Gly
            900                 905                 910
Leu Ile Gly Pro Leu Leu Val Cys His Thr Asn Thr Leu Asn Pro Ala
        915                 920                 925
His Gly Arg Gln Val Thr Val Gln Glu Phe Ala Leu Phe Phe Thr Ile
    930                 935                 940
Phe Asp Glu Thr Lys Ser Trp Tyr Phe Thr Glu Asn Met Glu Arg Asn
945                 950                 955                 960
Cys Arg Ala Pro Cys Asn Ile Gln Met Glu Asp Pro Thr Phe Lys Glu
                965                 970                 975
Asn Tyr Arg Phe His Ala Ile Asn Gly Tyr Ile Met Asp Thr Leu Pro
            980                 985                 990
Gly Leu Val Met Ala Gln Asp Gln Arg Ile Arg Trp Tyr Leu Leu Ser
        995                 1000                1005
Met Gly Ser Asn Glu Asn Ile His Ser Ile His Phe Ser Gly His Val
    1010                1015                1020
Phe Thr Val Arg Lys Lys Glu Glu Tyr Lys Met Ala Leu Tyr Asn Leu
1025                1030                1035                1040
Tyr Pro Gly Val Phe Glu Thr Val Glu Met Leu Pro Ser Lys Ala Gly
                1045                1050                1055

Ile Trp Arg Val Glu Cys Leu Ile Gly Glu His Leu His Ala Gly Met
            1060                1065                1070
Ser Thr Leu Phe Leu Val Tyr Ser Asn Lys Cys Gln Thr Pro Leu Gly
        1075                1080                1085
Met Ala Ser Gly His Ile Arg Asp Phe Gln Ile Thr Ala Ser Gly Gln
    1090                1095                1100
Tyr Gly Gln Trp Ala Pro Lys Leu Ala Arg Leu His Tyr Ser Gly Ser
1105                1110                1115                1120
Ile Asn Ala Trp Ser Thr Lys Glu Pro Phe Ser Trp Ile Lys Val Asp
                1125                1130                1135
Leu Leu Ala Pro Met Ile Ile His Gly Ile Lys Thr Gln Gly Ala Arg
            1140                1145                1150
Gln Lys Phe Ser Ser Leu Tyr Ile Ser Gln Phe Ile Ile Met Tyr Ser
        1155                1160                1165
Leu Asp Gly Lys Lys Trp Gln Thr Tyr Arg Gly Asn Ser Thr Gly Thr
    1170                1175                1180
Leu Met Val Phe Phe Gly Asn Val Asp Ser Ser Gly Ile Lys His Asn
1185                1190                1195                1200
Ile Phe Asn Pro Pro Ile Ile Ala Arg Tyr Ile Arg Leu His Pro Thr
                1205                1210                1215
His Tyr Ser Ile Arg Ser Thr Leu Arg Met Glu Leu Met Gly Cys Asp
            1220                1225                1230
Leu Asn Ser Cys Ser Met Pro Leu Gly Met Glu Ser Lys Ala Ile Ser
        1235                1240                1245
Asp Ala Gln Ile Thr Ala Ser Ser Tyr Phe Thr Asn Met Phe Ala Thr
    1250                1255                1260
Trp Ser Pro Ser Lys Ala Arg Leu His Leu Gln Gly Arg Ser Asn Ala
1265                1270                1275                1280
Trp Arg Pro Gln Val Asn Asn Pro Lys Glu Trp Leu Gln Val Asp Phe
                1285                1290                1295
Gln Lys Thr Met Lys Val Thr Gly Val Thr Thr Gln Gly Val Lys Ser
            1300                1305                1310
Leu Leu Thr Ser Met Tyr Val Lys Glu Phe Leu Ile Ser Ser Ser Gln
        1315                1320                1325
Asp Gly His Gln Trp Thr Leu Phe Phe Gln Asn Gly Lys Val Lys Val
    1330                1335                1340
Phe Gln Gly Asn Gln Asp Ser Phe Thr Pro Val Val Asn Ser Leu Asp
1345                1350                1355                1360
Pro Pro Leu Leu Thr Arg Tyr Leu Arg Ile His Pro Gln Ser Trp Val
                1365                1370                1375
His Gln Ile Ala Leu Arg Met Glu Val Leu Gly Cys Glu Ala Gln Asp
            1380                1385                1390
Leu Tyr

<210> 20
<211> 1404
<212> PRT
<213> Artificial Sequence

<220>
<221> SIGNAL
<222> 1..19

<220>
<223> v4 aa incl. signal sequence

<400> 20
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
            35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp

530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Pro Arg Ile Arg Ser
                725                 730                 735
Val Val Ala Gln Ser Val Glu Met Lys Lys Glu Asp Phe Asp Ile Tyr
            740                 745                 750
Asp Glu Asp Glu Asn Gln Ser Pro Arg Ser Phe Gln Lys Lys Thr Arg
        755                 760                 765
His Tyr Phe Ile Ala Ala Val Glu Arg Leu Trp Asp Tyr Gly Met Ser
    770                 775                 780
Ser Ser Pro His Val Leu Arg Asn Arg Ala Gln Ser Gly Ser Val Pro
785                 790                 795                 800
Gln Phe Lys Lys Val Val Phe Gln Glu Phe Thr Asp Gly Ser Phe Thr
                805                 810                 815
Gln Pro Leu Tyr Arg Gly Glu Leu Asn Glu His Leu Gly Leu Leu Gly
            820                 825                 830
Pro Tyr Ile Arg Ala Glu Val Glu Asp Asn Ile Met Val Thr Phe Arg
        835                 840                 845
Asn Gln Ala Ser Arg Pro Tyr Ser Phe Tyr Ser Ser Leu Ile Ser Tyr
    850                 855                 860
Glu Glu Asp Gln Arg Gln Gly Ala Glu Pro Arg Lys Asn Phe Val Lys
865                 870                 875                 880
Pro Asn Glu Thr Lys Thr Tyr Phe Trp Lys Val Gln His His Met Ala
                885                 890                 895
Pro Thr Lys Asp Glu Phe Asp Cys Lys Ala Trp Ala Tyr Phe Ser Asp
            900                 905                 910
Val Asp Leu Glu Lys Asp Val His Ser Gly Leu Ile Gly Pro Leu Leu
        915                 920                 925
Val Cys His Thr Asn Thr Leu Asn Pro Ala His Gly Arg Gln Val Thr
    930                 935                 940
Val Gln Glu Phe Ala Leu Phe Phe Thr Ile Phe Asp Glu Thr Lys Ser
945                 950                 955                 960
Trp Tyr Phe Thr Glu Asn Met Glu Arg Asn Cys Arg Ala Pro Cys Asn
                965                 970                 975
Ile Gln Met Glu Asp Pro Thr Phe Lys Glu Asn Tyr Arg Phe His Ala
            980                 985                 990
Ile Asn Gly Tyr Ile Met Asp Thr Leu Pro Gly Leu Val Met Ala Gln
        995                 1000                1005
Asp Gln Arg Ile Arg Trp Tyr Leu Leu Ser Met Gly Ser Asn Glu Asn
    1010                1015                1020
Ile His Ser Ile His Phe Ser Gly His Val Phe Thr Val Arg Lys Lys
1025                1030                1035                1040

Glu Glu Tyr Lys Met Ala Leu Tyr Asn Leu Tyr Pro Gly Val Phe Glu
                1045                1050                1055
Thr Val Glu Met Leu Pro Ser Lys Ala Gly Ile Trp Arg Val Glu Cys
            1060                1065                1070
Leu Ile Gly Glu His Leu His Ala Gly Met Ser Thr Leu Phe Leu Val
        1075                1080                1085
Tyr Ser Asn Lys Cys Gln Thr Pro Leu Gly Met Ala Ser Gly His Ile
    1090                1095                1100
Arg Asp Phe Gln Ile Thr Ala Ser Gly Gln Tyr Gly Gln Trp Ala Pro
1105                1110                1115                1120
Lys Leu Ala Arg Leu His Tyr Ser Gly Ser Ile Asn Ala Trp Ser Thr
                1125                1130                1135
Lys Glu Pro Phe Ser Trp Ile Lys Val Asp Leu Leu Ala Pro Met Ile
            1140                1145                1150
Ile His Gly Ile Lys Thr Gln Gly Ala Arg Gln Lys Phe Ser Ser Leu
        1155                1160                1165
Tyr Ile Ser Gln Phe Ile Ile Met Tyr Ser Leu Asp Gly Lys Lys Trp
    1170                1175                1180
Gln Thr Tyr Arg Gly Asn Ser Thr Gly Thr Leu Met Val Phe Phe Gly
1185                1190                1195                1200
Asn Val Asp Ser Ser Gly Ile Lys His Asn Ile Phe Asn Pro Pro Ile
                1205                1210                1215
Ile Ala Arg Tyr Ile Arg Leu His Pro Thr His Tyr Ser Ile Arg Ser
            1220                1225                1230
Thr Leu Arg Met Glu Leu Met Gly Cys Asp Leu Asn Ser Cys Ser Met
        1235                1240                1245
Pro Leu Gly Met Glu Ser Lys Ala Ile Ser Asp Ala Gln Ile Thr Ala
    1250                1255                1260
Ser Ser Tyr Phe Thr Asn Met Phe Ala Thr Trp Ser Pro Ser Lys Ala
1265                1270                1275                1280
Arg Leu His Leu Gln Gly Arg Ser Asn Ala Trp Arg Pro Gln Val Asn
                1285                1290                1295
Asn Pro Lys Glu Trp Leu Gln Val Asp Phe Gln Lys Thr Met Lys Val
            1300                1305                1310
Thr Gly Val Thr Thr Gln Gly Val Lys Ser Leu Leu Thr Ser Met Tyr
        1315                1320                1325
Val Lys Glu Phe Leu Ile Ser Ser Ser Gln Asp Gly His Gln Trp Thr
    1330                1335                1340
Leu Phe Phe Gln Asn Gly Lys Val Lys Val Phe Gln Gly Asn Gln Asp
1345                1350                1355                1360
Ser Phe Thr Pro Val Val Asn Ser Leu Asp Pro Pro Leu Leu Thr Arg
                1365                1370                1375
Tyr Leu Arg Ile His Pro Gln Ser Trp Val His Gln Ile Ala Leu Arg
            1380                1385                1390
Met Glu Val Leu Gly Cys Glu Ala Gln Asp Leu Tyr
        1395                1400

<210> 21
<211> 1438
<212> PRT
<213> Artificial Sequence

<220>
<221> SIGNAL
<222> 1..19

<220>
<223> V5 aa incl. signal sequence

<400> 21
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe

515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Ile
        755                 760                 765
Asp Tyr Asp Asp Thr Ile Ser Val Glu Met Lys Lys Glu Asp Phe Asp
    770                 775                 780
Ile Tyr Asp Glu Asp Glu Asn Gln Ser Pro Arg Ser Phe Gln Lys Lys
785                 790                 795                 800
Thr Arg His Tyr Phe Ile Ala Ala Val Glu Arg Leu Trp Asp Tyr Gly
                805                 810                 815
Met Ser Ser Ser Pro His Val Leu Arg Asn Arg Ala Gln Ser Gly Ser
            820                 825                 830
Val Pro Gln Phe Lys Lys Val Val Phe Gln Glu Phe Thr Asp Gly Ser
        835                 840                 845
Phe Thr Gln Pro Leu Tyr Arg Gly Glu Leu Asn Glu His Leu Gly Leu
    850                 855                 860
Leu Gly Pro Tyr Ile Arg Ala Glu Val Glu Asp Asn Ile Met Val Thr
865                 870                 875                 880
Phe Arg Asn Gln Ala Ser Arg Pro Tyr Ser Phe Tyr Ser Ser Leu Ile
                885                 890                 895
Ser Tyr Glu Glu Asp Gln Arg Gln Gly Ala Glu Pro Arg Lys Asn Phe
            900                 905                 910
Val Lys Pro Asn Glu Thr Lys Thr Tyr Phe Trp Lys Val Gln His His
        915                 920                 925
Met Ala Pro Thr Lys Asp Glu Phe Asp Cys Lys Ala Trp Ala Tyr Phe
    930                 935                 940
Ser Asp Val Asp Leu Glu Lys Asp Val His Ser Gly Leu Ile Gly Pro
945                 950                 955                 960
Leu Leu Val Cys His Thr Asn Thr Leu Asn Pro Ala His Gly Arg Gln
                965                 970                 975
Val Thr Val Gln Glu Phe Ala Leu Phe Phe Thr Ile Phe Asp Glu Thr
            980                 985                 990
Lys Ser Trp Tyr Phe Thr Glu Asn Met Glu Arg Asn Cys Arg Ala Pro
        995                 1000                1005
Cys Asn Ile Gln Met Glu Asp Pro Thr Phe Lys Glu Asn Tyr Arg Phe
    1010                1015                1020

His Ala Ile Asn Gly Tyr Ile Met Asp Thr Leu Pro Gly Leu Val Met
1025                1030                1035                1040
Ala Gln Asp Gln Arg Ile Arg Trp Tyr Leu Leu Ser Met Gly Ser Asn
                1045                1050                1055
Glu Asn Ile His Ser Ile His Phe Ser Gly His Val Phe Thr Val Arg
            1060                1065                1070
Lys Lys Glu Glu Tyr Lys Met Ala Leu Tyr Asn Leu Tyr Pro Gly Val
        1075                1080                1085
Phe Glu Thr Val Glu Met Leu Pro Ser Lys Ala Gly Ile Trp Arg Val
    1090                1095                1100
Glu Cys Leu Ile Gly Glu His Leu His Ala Gly Met Ser Thr Leu Phe
1105                1110                1115                1120
Leu Val Tyr Ser Asn Lys Cys Gln Thr Pro Leu Gly Met Ala Ser Gly
                1125                1130                1135
His Ile Arg Asp Phe Gln Ile Thr Ala Ser Gly Gln Tyr Gly Gln Trp
            1140                1145                1150
Ala Pro Lys Leu Ala Arg Leu His Tyr Ser Gly Ser Ile Asn Ala Trp
        1155                1160                1165
Ser Thr Lys Glu Pro Phe Ser Trp Ile Lys Val Asp Leu Leu Ala Pro
    1170                1175                1180
Met Ile Ile His Gly Ile Lys Thr Gln Gly Ala Arg Gln Lys Phe Ser
1185                1190                1195                1200
Ser Leu Tyr Ile Ser Gln Phe Ile Ile Met Tyr Ser Leu Asp Gly Lys
                1205                1210                1215
Lys Trp Gln Thr Tyr Arg Gly Asn Ser Thr Gly Thr Leu Met Val Phe
            1220                1225                1230
Phe Gly Asn Val Asp Ser Ser Gly Ile Lys His Asn Ile Phe Asn Pro
        1235                1240                1245
Pro Ile Ile Ala Arg Tyr Ile Arg Leu His Pro Thr His Tyr Ser Ile
    1250                1255                1260
Arg Ser Thr Leu Arg Met Glu Leu Met Gly Cys Asp Leu Asn Ser Cys
1265                1270                1275                1280
Ser Met Pro Leu Gly Met Glu Ser Lys Ala Ile Ser Asp Ala Gln Ile
                1285                1290                1295
Thr Ala Ser Ser Tyr Phe Thr Asn Met Phe Ala Thr Trp Ser Pro Ser
            1300                1305                1310
Lys Ala Arg Leu His Leu Gln Gly Arg Ser Asn Ala Trp Arg Pro Gln
        1315                1320                1325
Val Asn Asn Pro Lys Glu Trp Leu Gln Val Asp Phe Gln Lys Thr Met
    1330                1335                1340
Lys Val Thr Gly Val Thr Thr Gln Gly Val Lys Ser Leu Leu Thr Ser
1345                1350                1355                1360
Met Tyr Val Lys Glu Phe Leu Ile Ser Ser Ser Gln Asp Gly His Gln
                1365                1370                1375
Trp Thr Leu Phe Phe Gln Asn Gly Lys Val Lys Val Phe Gln Gly Asn
            1380                1385                1390
Gln Asp Ser Phe Thr Pro Val Val Asn Ser Leu Asp Pro Pro Leu Leu
        1395                1400                1405
Thr Arg Tyr Leu Arg Ile His Pro Gln Ser Trp Val His Gln Ile Ala
    1410                1415                1420
Leu Arg Met Glu Val Leu Gly Cys Glu Ala Gln Asp Leu Tyr
1425                1430                1435

<210> 22
<211> 1437
<212> PRT
<213> Artificial Sequence

<220>
<221> SIGNAL
<222> 1..19

<220>
<223> V6 aa incl. signal sequence

<400> 22
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu

465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Ile Asp
        755                 760                 765
Tyr Asp Asp Thr Ile Ser Val Glu Met Lys Lys Glu Asp Phe Asp Ile
    770                 775                 780
Tyr Asp Glu Asp Glu Asn Gln Ser Pro Arg Ser Phe Gln Lys Lys Thr
785                 790                 795                 800
Arg His Tyr Phe Ile Ala Ala Val Glu Arg Leu Trp Asp Tyr Gly Met
                805                 810                 815
Ser Ser Ser Pro His Val Leu Arg Asn Arg Ala Gln Ser Gly Ser Val
            820                 825                 830
Pro Gln Phe Lys Lys Val Val Phe Gln Glu Phe Thr Asp Gly Ser Phe
        835                 840                 845
Thr Gln Pro Leu Tyr Arg Gly Glu Leu Asn Glu His Leu Gly Leu Leu
    850                 855                 860
Gly Pro Tyr Ile Arg Ala Glu Val Glu Asp Asn Ile Met Val Thr Phe
865                 870                 875                 880
Arg Asn Gln Ala Ser Arg Pro Tyr Ser Phe Tyr Ser Ser Leu Ile Ser
                885                 890                 895
Tyr Glu Glu Asp Gln Arg Gln Gly Ala Glu Pro Arg Lys Asn Phe Val
            900                 905                 910
Lys Pro Asn Glu Thr Lys Thr Tyr Phe Trp Lys Val Gln His His Met
        915                 920                 925
Ala Pro Thr Lys Asp Glu Phe Asp Cys Lys Ala Trp Ala Tyr Phe Ser
    930                 935                 940
Asp Val Asp Leu Glu Lys Asp Val His Ser Gly Leu Ile Gly Pro Leu
945                 950                 955                 960
Leu Val Cys His Thr Asn Thr Leu Asn Pro Ala His Gly Arg Gln Val
                965                 970                 975

Thr Val Gln Glu Phe Ala Leu Phe Phe Thr Ile Phe Asp Glu Thr Lys
            980                 985                 990
Ser Trp Tyr Phe Thr Glu Asn Met Glu Arg Asn Cys Arg Ala Pro Cys
        995                 1000                1005
Asn Ile Gln Met Glu Asp Pro Thr Phe Lys Glu Asn Tyr Arg Phe His
    1010                1015                1020
Ala Ile Asn Gly Tyr Ile Met Asp Thr Leu Pro Gly Leu Val Met Ala
1025                1030                1035                1040
Gln Asp Gln Arg Ile Arg Trp Tyr Leu Leu Ser Met Gly Ser Asn Glu
                1045                1050                1055
Asn Ile His Ser Ile His Phe Ser Gly His Val Phe Thr Val Arg Lys
            1060                1065                1070
Lys Glu Glu Tyr Lys Met Ala Leu Tyr Asn Leu Tyr Pro Gly Val Phe
        1075                1080                1085
Glu Thr Val Glu Met Leu Pro Ser Lys Ala Gly Ile Trp Arg Val Glu
    1090                1095                1100
Cys Leu Ile Gly Glu His Leu His Ala Gly Met Ser Thr Leu Phe Leu
1105                1110                1115                1120
Val Tyr Ser Asn Lys Cys Gln Thr Pro Leu Gly Met Ala Ser Gly His
                1125                1130                1135
Ile Arg Asp Phe Gln Ile Thr Ala Ser Gly Gln Tyr Gly Gln Trp Ala
            1140                1145                1150
Pro Lys Leu Ala Arg Leu His Tyr Ser Gly Ser Ile Asn Ala Trp Ser
        1155                1160                1165
Thr Lys Glu Pro Phe Ser Trp Ile Lys Val Asp Leu Leu Ala Pro Met
    1170                1175                1180
Ile Ile His Gly Ile Lys Thr Gln Gly Ala Arg Gln Lys Phe Ser Ser
1185                1190                1195                1200
Leu Tyr Ile Ser Gln Phe Ile Ile Met Tyr Ser Leu Asp Gly Lys Lys
                1205                1210                1215
Trp Gln Thr Tyr Arg Gly Asn Ser Thr Gly Thr Leu Met Val Phe Phe
            1220                1225                1230
Gly Asn Val Asp Ser Ser Gly Ile Lys His Asn Ile Phe Asn Pro Pro
        1235                1240                1245
Ile Ile Ala Arg Tyr Ile Arg Leu His Pro Thr His Tyr Ser Ile Arg
    1250                1255                1260
Ser Thr Leu Arg Met Glu Leu Met Gly Cys Asp Leu Asn Ser Cys Ser
1265                1270                1275                1280
Met Pro Leu Gly Met Glu Ser Lys Ala Ile Ser Asp Ala Gln Ile Thr
                1285                1290                1295
Ala Ser Ser Tyr Phe Thr Asn Met Phe Ala Thr Trp Ser Pro Ser Lys
            1300                1305                1310
Ala Arg Leu His Leu Gln Gly Arg Ser Asn Ala Trp Arg Pro Gln Val
        1315                1320                1325
Asn Asn Pro Lys Glu Trp Leu Gln Val Asp Phe Gln Lys Thr Met Lys
    1330                1335                1340
Val Thr Gly Val Thr Thr Gln Gly Val Lys Ser Leu Leu Thr Ser Met
1345                1350                1355                1360
Tyr Val Lys Glu Phe Leu Ile Ser Ser Ser Gln Asp Gly His Gln Trp
                1365                1370                1375
Thr Leu Phe Phe Gln Asn Gly Lys Val Lys Val Phe Gln Gly Asn Gln
            1380                1385                1390
Asp Ser Phe Thr Pro Val Val Asn Ser Leu Asp Pro Pro Leu Leu Thr
        1395                1400                1405
Arg Tyr Leu Arg Ile His Pro Gln Ser Trp Val His Gln Ile Ala Leu
    1410                1415                1420
Arg Met Glu Val Leu Gly Cys Glu Ala Gln Asp Leu Tyr
1425                1430                1435

<210> 23
<211> 1429
<212> PRT
<213> Artificial Sequence

<220>
<221> SIGNAL
<222> 1..19

<220>
<223> V7 aa incl. signal sequence

<400> 23
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn

            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Glu
        755                 760                 765
Met Lys Lys Glu Asp Phe Asp Ile Tyr Asp Glu Asp Glu Asn Gln Ser
    770                 775                 780
Pro Arg Ser Phe Gln Lys Lys Thr Arg His Tyr Phe Ile Ala Ala Val
785                 790                 795                 800
Glu Arg Leu Trp Asp Tyr Gly Met Ser Ser Ser Pro His Val Leu Arg
                805                 810                 815
Asn Arg Ala Gln Ser Gly Ser Val Pro Gln Phe Lys Lys Val Val Phe
            820                 825                 830
Gln Glu Phe Thr Asp Gly Ser Phe Thr Gln Pro Leu Tyr Arg Gly Glu
        835                 840                 845
Leu Asn Glu His Leu Gly Leu Leu Gly Pro Tyr Ile Arg Ala Glu Val
    850                 855                 860
Glu Asp Asn Ile Met Val Thr Phe Arg Asn Gln Ala Ser Arg Pro Tyr
865                 870                 875                 880
Ser Phe Tyr Ser Ser Leu Ile Ser Tyr Glu Glu Asp Gln Arg Gln Gly
                885                 890                 895
Ala Glu Pro Arg Lys Asn Phe Val Lys Pro Asn Glu Thr Lys Thr Tyr
            900                 905                 910
Phe Trp Lys Val Gln His His Met Ala Pro Thr Lys Asp Glu Phe Asp
        915                 920                 925

Cys Lys Ala Trp Ala Tyr Phe Ser Asp Val Asp Leu Glu Lys Asp Val
    930                 935                 940
His Ser Gly Leu Ile Gly Pro Leu Leu Val Cys His Thr Asn Thr Leu
945                 950                 955                 960
Asn Pro Ala His Gly Arg Gln Val Thr Val Gln Glu Phe Ala Leu Phe
                965                 970                 975
Phe Thr Ile Phe Asp Glu Thr Lys Ser Trp Tyr Phe Thr Glu Asn Met
            980                 985                 990
Glu Arg Asn Cys Arg Ala Pro Cys Asn Ile Gln Met Glu Asp Pro Thr
        995                 1000                1005
Phe Lys Glu Asn Tyr Arg Phe His Ala Ile Asn Gly Tyr Ile Met Asp
    1010                1015                1020
Thr Leu Pro Gly Leu Val Met Ala Gln Asp Gln Arg Ile Arg Trp Tyr
1025                1030                1035                1040
Leu Leu Ser Met Gly Ser Asn Glu Asn Ile His Ser Ile His Phe Ser
                1045                1050                1055
Gly His Val Phe Thr Val Arg Lys Lys Glu Glu Tyr Lys Met Ala Leu
            1060                1065                1070
Tyr Asn Leu Tyr Pro Gly Val Phe Glu Thr Val Glu Met Leu Pro Ser
        1075                1080                1085
Lys Ala Gly Ile Trp Arg Val Glu Cys Leu Ile Gly Glu His Leu His
    1090                1095                1100
Ala Gly Met Ser Thr Leu Phe Leu Val Tyr Ser Asn Lys Cys Gln Thr
1105                1110                1115                1120
Pro Leu Gly Met Ala Ser Gly His Ile Arg Asp Phe Gln Ile Thr Ala
                1125                1130                1135
Ser Gly Gln Tyr Gly Gln Trp Ala Pro Lys Leu Ala Arg Leu His Tyr
            1140                1145                1150
Ser Gly Ser Ile Asn Ala Trp Ser Thr Lys Glu Pro Phe Ser Trp Ile
        1155                1160                1165
Lys Val Asp Leu Leu Ala Pro Met Ile Ile His Gly Ile Lys Thr Gln
    1170                1175                1180
Gly Ala Arg Gln Lys Phe Ser Ser Leu Tyr Ile Ser Gln Phe Ile Ile
1185                1190                1195                1200
Met Tyr Ser Leu Asp Gly Lys Lys Trp Gln Thr Tyr Arg Gly Asn Ser
                1205                1210                1215
Thr Gly Thr Leu Met Val Phe Phe Gly Asn Val Asp Ser Ser Gly Ile
            1220                1225                1230
Lys His Asn Ile Phe Asn Pro Pro Ile Ile Ala Arg Tyr Ile Arg Leu
        1235                1240                1245
His Pro Thr His Tyr Ser Ile Arg Ser Thr Leu Arg Met Glu Leu Met
    1250                1255                1260
Gly Cys Asp Leu Asn Ser Cys Ser Met Pro Leu Gly Met Glu Ser Lys
1265                1270                1275                1280
Ala Ile Ser Asp Ala Gln Ile Thr Ala Ser Ser Tyr Phe Thr Asn Met
                1285                1290                1295
Phe Ala Thr Trp Ser Pro Ser Lys Ala Arg Leu His Leu Gln Gly Arg
            1300                1305                1310
Ser Asn Ala Trp Arg Pro Gln Val Asn Asn Pro Lys Glu Trp Leu Gln
        1315                1320                1325
Val Asp Phe Gln Lys Thr Met Lys Val Thr Gly Val Thr Thr Gln Gly
    1330                1335                1340
Val Lys Ser Leu Leu Thr Ser Met Tyr Val Lys Glu Phe Leu Ile Ser
1345                1350                1355                1360
Ser Ser Gln Asp Gly His Gln Trp Thr Leu Phe Phe Gln Asn Gly Lys
                1365                1370                1375
Val Lys Val Phe Gln Gly Asn Gln Asp Ser Phe Thr Pro Val Val Asn
            1380                1385                1390
Ser Leu Asp Pro Pro Leu Leu Thr Arg Tyr Leu Arg Ile His Pro Gln
        1395                1400                1405
Ser Trp Val His Gln Ile Ala Leu Arg Met Glu Val Leu Gly Cys Glu
    1410                1415                1420
Ala Gln Asp Leu Tyr

1425

<210> 24
<211> 4335
<212> DNA
<213> Artificial Sequence


<220>
<223> V0 na incl. signal sequence without stop codon

<220>
<221> sig_peptide
<222> 1..57

<400> 24
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt gctgcagagc gaccaggaag agatcgacta cgacgacacc 2340

atcagcgtcg agatgaagaa agaggacttc gacatctacg acgaggacga gaaccagagc 2400

ccccggtcct tccagaagaa aacccggcac tactttatcg ccgccgtgga acggctgtgg 2460

gactacggca tgagcagcag cccccacgtg ctgcggaata gagcccagag cggcagcgtg 2520

ccccagttca agaaagtggt gttccaggag ttcaccgacg gcagcttcac ccagcctctg 2580

taccggggcg agctgaacga gcacctgggc ctgctgggcc cctacatccg ggccgaggtg 2640

gaagataaca tcatggtcac cttccggaac caggccagcc ggccctacag cttctacagc 2700

agcctgatct cctacgaaga ggaccagcgg cagggcgccg agccccggaa gaacttcgtg 2760

aagcccaacg agacaaagac ctacttctgg aaggtgcagc accacatggc ccccaccaag 2820

gacgaattcg actgcaaggc ctgggcctac ttcagcgacg tggacctgga aaaggacgtg 2880

cacagcggcc tgatcggccc cctgctcgtg tgccacacca acaccctgaa ccccgcccac 2940

ggccggcagg tcacagtgca ggaatttgcc ctgttcttca ccatcttcga cgagactaag 3000

agctggtact tcaccgagaa catggaacgg aactgcagag ccccctgcaa catccagatg 3060

gaagatccca ccttcaaaga gaactaccgg ttccacgcca tcaatggcta catcatggac 3120

accctgcccg gcctggtcat ggcccaggac cagagaatcc ggtggtatct gctgagcatg 3180

ggcagcaacg agaacatcca cagcatccac ttcagcggcc acgtgttcac cgtgcggaag 3240

aaagaagagt acaagatggc cctgtacaac ctgtaccccg gcgtgttcga aacagtggaa 3300

atgctgccca gcaaggccgg catctggcgg gtggaatgtc tgatcggcga gcatctgcac 3360

gccggcatgt ccaccctgtt tctggtgtac agcaacaagt gccagacccc cctgggcatg 3420

gccagcggcc acatccggga tttccagatc accgcctccg gccagtacgg ccagtgggcc 3480

cctaaactgg cccggctgca ctacagcggc agcatcaacg cctggtccac caaagagccc 3540

ttcagctgga tcaaggtgga cctgctggcc cccatgatta tccacggcat caagacacag 3600

ggcgccagac agaagttcag cagcctgtac atcagccagt tcatcatcat gtacagcctg 3660

gatggcaaga agtggcagac ctaccggggc aacagcaccg gcaccctgat ggtgttcttc 3720

ggcaacgtgg acagcagcgg catcaagcac aacatcttca acccccccat cattgcccgg 3780

tacatccggc tgcaccccac ccactacagc atccggtcca ccctgcggat ggaactgatg 3840

ggctgcgacc tgaacagctg ctccatgcct ctgggcatgg aaagcaaggc catcagcgac 3900

gcccagatca cagccagcag ctacttcacc aacatgttcg ccacctggtc cccatccaag 3960

gccagactgc atctgcaggg cagaagcaat gcctggcggc ctcaggtcaa caaccccaaa 4020

gaatggctcc aggtggactt ccagaaaacc atgaaggtca caggcgtgac cacccagggc 4080

gtgaagtccc tgctgacctc tatgtacgtg aaagagttcc tgatctccag cagccaggac 4140

ggccaccagt ggaccctgtt ctttcagaac ggcaaagtga aagtgttcca gggcaaccag 4200

gactccttca cccccgtggt caactccctg gaccccccac tgctgaccag atacctgaga 4260

atccaccccc agagctgggt gcaccagatc gccctgagaa tggaagtgct gggatgcgag 4320

gcccaggatc tgtac 4335

<210> 25
<211> 4314
<212> DNA
<213> Artificial Sequence

<220>
<223> V5 na incl. signal sequence without stop codon

<220>
<221> sig_peptide
<222> 1..57

<400> 25
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt gatcgactac gacgacacca tcagcgtcga gatgaagaaa 2340

gaggacttcg acatctacga cgaggacgag aaccagagcc cccggtcctt ccagaagaaa 2400

acccggcact actttatcgc cgccgtggaa cggctgtggg actacggcat gagcagcagc 2460

ccccacgtgc tgcggaatag agcccagagc ggcagcgtgc cccagttcaa gaaagtggtg 2520

ttccaggagt tcaccgacgg cagcttcacc cagcctctgt accggggcga gctgaacgag 2580

cacctgggcc tgctgggccc ctacatccgg gccgaggtgg aagataacat catggtcacc 2640

ttccggaacc aggccagccg gccctacagc ttctacagca gcctgatctc ctacgaagag 2700

gaccagcggc agggcgccga gccccggaag aacttcgtga agcccaacga gacaaagacc 2760

tacttctgga aggtgcagca ccacatggcc cccaccaagg acgaattcga ctgcaaggcc 2820

tgggcctact tcagcgacgt ggacctggaa aaggacgtgc acagcggcct gatcggcccc 2880

ctgctcgtgt gccacaccaa caccctgaac cccgcccacg gccggcaggt cacagtgcag 2940

gaatttgccc tgttcttcac catcttcgac gagactaaga gctggtactt caccgagaac 3000

atggaacgga actgcagagc cccctgcaac atccagatgg aagatcccac cttcaaagag 3060

aactaccggt tccacgccat caatggctac atcatggaca ccctgcccgg cctggtcatg 3120

gcccaggacc agagaatccg gtggtatctg ctgagcatgg gcagcaacga gaacatccac 3180

agcatccact tcagcggcca cgtgttcacc gtgcggaaga aagaagagta caagatggcc 3240

ctgtacaacc tgtaccccgg cgtgttcgag acagtggaaa tgctgcccag caaggccggc 3300

atctggcggg tggaatgtct gatcggcgag catctgcacg ccggcatgtc caccctgttt 3360

ctggtgtaca gcaacaagtg ccagaccccc ctgggcatgg ccagcggcca catccgggat 3420

ttccagatca ccgcctccgg ccagtacggc cagtgggccc ctaaactggc ccggctgcac 3480

tacagcggca gcatcaacgc ctggtccacc aaagagccct tcagctggat caaggtggac 3540

ctgctggccc ccatgattat ccacggcatc aagacacagg gcgccagaca gaagttcagc 3600

agcctgtaca tcagccagtt catcatcatg tacagcctgg atggcaagaa gtggcagacc 3660

taccggggca acagcaccgg caccctgatg gtgttcttcg gcaacgtgga cagcagcggc 3720

atcaagcaca acatcttcaa cccccccatc attgcccggt acatccggct gcaccccacc 3780

cactacagca tccggtccac cctgcggatg gaactgatgg gctgcgacct gaacagctgc 3840

tccatgcctc tgggcatgga aagcaaggcc atcagcgacg cccagatcac agccagcagc 3900

tacttcacca acatgttcgc cacctggtcc ccatccaagg ccagactgca tctgcagggc 3960

agaagcaatg cctggcggcc tcaggtcaac aaccccaaag aatggctcca ggtggacttc 4020

cagaaaacca tgaaggtcac aggcgtgacc acccagggcg tgaagtccct gctgacctct 4080

atgtacgtga aagagttcct gatctccagc agccaggacg gccaccagtg gaccctgttc 4140

tttcagaacg gcaaagtgaa agtgttccag ggcaaccagg actccttcac ccccgtggtc 4200

aactccctgg accccccact gctgaccaga tacctgagaa tccaccccca gagctgggtg 4260

caccagatcg ccctgagaat ggaagtgctg ggatgcgagg cccaggatct gtac 4314

<210> 26
<211> 4311
<212> DNA
<213> Artificial Sequence

<220>
<223> V6 na incl. signal sequence withot stop codon

<220>
<221> sig_peptide
<222> 1..57

<400> 26
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccat cgactacgac gacaccatca gcgtcgagat gaagaaagag 2340

gacttcgaca tctacgacga ggacgagaac cagagccccc ggtccttcca gaagaaaacc 2400

cggcactact ttatcgccgc cgtggaacgg ctgtgggact acggcatgag cagcagcccc 2460

cacgtgctgc ggaatagagc ccagagcggc agcgtgcccc agttcaagaa agtggtgttc 2520

caggagttca ccgacggcag cttcacccag cctctgtacc ggggcgagct gaacgagcac 2580

ctgggcctgc tgggccccta catccgggcc gaggtggaag ataacatcat ggtcaccttc 2640

cggaaccagg ccagccggcc ctacagcttc tacagcagcc tgatctccta cgaagaggac 2700

cagcggcagg gcgccgagcc ccggaagaac ttcgtgaagc ccaacgagac aaagacctac 2760

ttctggaagg tgcagcacca catggccccc accaaggacg aattcgactg caaggcctgg 2820

gcctacttca gcgacgtgga cctggaaaag gacgtgcaca gcggcctgat cggccccctg 2880

ctcgtgtgcc acaccaacac cctgaacccc gcccacggcc ggcaggtcac agtgcaggaa 2940

tttgccctgt tcttcaccat cttcgacgag actaagagct ggtacttcac cgagaacatg 3000

gaacggaact gcagagcccc ctgcaacatc cagatggaag atcccacctt caaagagaac 3060

taccggttcc acgccatcaa tggctacatc atggacaccc tgcccggcct ggtcatggcc 3120

caggaccaga gaatccggtg gtatctgctg agcatgggca gcaacgagaa catccacagc 3180

atccacttca gcggccacgt gttcaccgtg cggaagaaag aagagtacaa gatggccctg 3240

tacaacctgt accccggcgt gttcgagaca gtggaaatgc tgcccagcaa ggccggcatc 3300

tggcgggtgg aatgtctgat cggcgagcat ctgcacgccg gcatgtccac cctgtttctg 3360

gtgtacagca acaagtgcca gacccccctg ggcatggcca gcggccacat ccgggatttc 3420

cagatcaccg cctccggcca gtacggccag tgggccccta aactggcccg gctgcactac 3480

agcggcagca tcaacgcctg gtccaccaaa gagcccttca gctggatcaa ggtggacctg 3540

ctggccccca tgattatcca cggcatcaag acacagggcg ccagacagaa gttcagcagc 3600

ctgtacatca gccagttcat catcatgtac agcctggatg gcaagaagtg gcagacctac 3660

cggggcaaca gcaccggcac cctgatggtg ttcttcggca acgtggacag cagcggcatc 3720

aagcacaaca tcttcaaccc ccccatcatt gcccggtaca tccggctgca ccccacccac 3780

tacagcatcc ggtccaccct gcggatggaa ctgatgggct gcgacctgaa cagctgctcc 3840

atgcctctgg gcatggaaag caaggccatc agcgacgccc agatcacagc cagcagctac 3900

ttcaccaaca tgttcgccac ctggtcccca tccaaggcca gactgcatct gcagggcaga 3960

agcaatgcct ggcggcctca ggtcaacaac cccaaagaat ggctccaggt ggacttccag 4020

aaaaccatga aggtcacagg cgtgaccacc cagggcgtga agtccctgct gacctctatg 4080

tacgtgaaag agttcctgat ctccagcagc caggacggcc accagtggac cctgttcttt 4140

cagaacggca aagtgaaagt gttccagggc aaccaggact ccttcacccc cgtggtcaac 4200

tccctggacc ccccactgct gaccagatac ctgagaatcc acccccagag ctgggtgcac 4260

cagatcgccc tgagaatgga agtgctggga tgcgaggccc aggatctgta c 4311

<210> 27
<211> 4287
<212> DNA
<213> Artificial Sequence

<220>
<223> V7 na incl. signal sequence without stop codon

<220>
<221> sig_peptide
<222> 1..57

<400> 27
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt ggagatgaag aaagaggact tcgacatcta cgacgaggac 2340

gagaaccaga gcccccggtc cttccagaag aaaacccggc actactttat cgccgccgtg 2400

gaacggctgt gggactacgg catgagcagc agcccccacg tgctgcggaa tagagcccag 2460

agcggcagcg tgccccagtt caagaaagtg gtgttccagg agttcaccga cggcagcttc 2520

acccagcctc tgtaccgggg cgagctgaac gagcacctgg gcctgctggg cccctacatc 2580

cgggccgagg tggaagataa catcatggtc accttccgga accaggccag ccggccctac 2640

agcttctaca gcagcctgat ctcctacgaa gaggaccagc ggcagggcgc cgagccccgg 2700

aagaacttcg tgaagcccaa cgagacaaag acctacttct ggaaggtgca gcaccacatg 2760

gcccccacca aggacgaatt cgactgcaag gcctgggcct acttcagcga cgtggacctg 2820

gaaaaggacg tgcacagcgg cctgatcggc cccctgctcg tgtgccacac caacaccctg 2880

aaccccgccc acggccggca ggtcacagtg caggaatttg ccctgttctt caccatcttc 2940

gacgagacta agagctggta cttcaccgag aacatggaac ggaactgcag agccccctgc 3000

aacatccaga tggaagatcc caccttcaaa gagaactacc ggttccacgc catcaatggc 3060

tacatcatgg acaccctgcc cggcctggtc atggcccagg accagagaat ccggtggtat 3120

ctgctgagca tgggcagcaa cgagaacatc cacagcatcc acttcagcgg ccacgtgttc 3180

accgtgcgga agaaagaaga gtacaagatg gccctgtaca acctgtaccc cggcgtgttc 3240

gagacagtgg aaatgctgcc cagcaaggcc ggcatctggc gggtggaatg tctgatcggc 3300

gagcatctgc acgccggcat gtccaccctg tttctggtgt acagcaacaa gtgccagacc 3360

cccctgggca tggccagcgg ccacatccgg gatttccaga tcaccgcctc cggccagtac 3420

ggccagtggg cccctaaact ggcccggctg cactacagcg gcagcatcaa cgcctggtcc 3480

accaaagagc ccttcagctg gatcaaggtg gacctgctgg cccccatgat tatccacggc 3540

atcaagacac agggcgccag acagaagttc agcagcctgt acatcagcca gttcatcatc 3600

atgtacagcc tggatggcaa gaagtggcag acctaccggg gcaacagcac cggcaccctg 3660

atggtgttct tcggcaacgt ggacagcagc ggcatcaagc acaacatctt caaccccccc 3720

atcattgccc ggtacatccg gctgcacccc acccactaca gcatccggtc caccctgcgg 3780

atggaactga tgggctgcga cctgaacagc tgctccatgc ctctgggcat ggaaagcaag 3840

gccatcagcg acgcccagat cacagccagc agctacttca ccaacatgtt cgccacctgg 3900

tccccatcca aggccagact gcatctgcag ggcagaagca atgcctggcg gcctcaggtc 3960

aacaacccca aagaatggct ccaggtggac ttccagaaaa ccatgaaggt cacaggcgtg 4020

accacccagg gcgtgaagtc cctgctgacc tctatgtacg tgaaagagtt cctgatctcc 4080

agcagccagg acggccacca gtggaccctg ttctttcaga acggcaaagt gaaagtgttc     4140

cagggcaacc aggactcctt cacccccgtg gtcaactccc tggacccccc actgctgacc     4200

agatacctga gaatccaccc ccagagctgg gtgcaccaga tcgccctgag aatggaagtg     4260

ctgggatgcg aggcccagga tctgtac                                         4287

<210> 28
<211> 1457
<212> PRT
<213> Artificial Sequence

<220>
<221> SIGNAL
<222> 1..19

<220>
<221> SIGNAL
<222> 1..19

<220>
<223> AC-6rs-REF aa incl. signal

<400> 28
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile

275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Lys Arg His Gln Arg Glu Ile Thr Arg Thr Thr Leu Gln Ser Asp Gln
    770                 775                 780

Glu Glu Ile Asp Tyr Asp Asp Thr Ile Ser Val Glu Met Lys Lys Glu
785                 790                 795                 800
Asp Phe Asp Ile Tyr Asp Glu Asp Glu Asn Gln Ser Pro Arg Ser Phe
                805                 810                 815
Gln Lys Lys Thr Arg His Tyr Phe Ile Ala Ala Val Glu Arg Leu Trp
            820                 825                 830
Asp Tyr Gly Met Ser Ser Ser Pro His Val Leu Arg Asn Arg Ala Gln
        835                 840                 845
Ser Gly Ser Val Pro Gln Phe Lys Lys Val Val Phe Gln Glu Phe Thr
    850                 855                 860
Asp Gly Ser Phe Thr Gln Pro Leu Tyr Arg Gly Glu Leu Asn Glu His
865                 870                 875                 880
Leu Gly Leu Leu Gly Pro Tyr Ile Arg Ala Glu Val Glu Asp Asn Ile
                885                 890                 895
Met Val Thr Phe Arg Asn Gln Ala Ser Arg Pro Tyr Ser Phe Tyr Ser
            900                 905                 910
Ser Leu Ile Ser Tyr Glu Glu Asp Gln Arg Gln Gly Ala Glu Pro Arg
        915                 920                 925
Lys Asn Phe Val Lys Pro Asn Glu Thr Lys Thr Tyr Phe Trp Lys Val
    930                 935                 940
Gln His His Met Ala Pro Thr Lys Asp Glu Phe Asp Cys Lys Ala Trp
945                 950                 955                 960
Ala Tyr Phe Ser Asp Val Asp Leu Glu Lys Asp Val His Ser Gly Leu
                965                 970                 975
Ile Gly Pro Leu Leu Val Cys His Thr Asn Thr Leu Asn Pro Ala His
            980                 985                 990
Gly Arg Gln Val Thr Val Gln Glu Phe Ala Leu Phe Phe Thr Ile Phe
        995                 1000                1005
Asp Glu Thr Lys Ser Trp Tyr Phe Thr Glu Asn Met Glu Arg Asn Cys
    1010                1015                1020
Arg Ala Pro Cys Asn Ile Gln Met Glu Asp Pro Thr Phe Lys Glu Asn
1025                1030                1035                1040
Tyr Arg Phe His Ala Ile Asn Gly Tyr Ile Met Asp Thr Leu Pro Gly
                1045                1050                1055
Leu Val Met Ala Gln Asp Gln Arg Ile Arg Trp Tyr Leu Leu Ser Met
            1060                1065                1070
Gly Ser Asn Glu Asn Ile His Ser Ile His Phe Ser Gly His Val Phe
        1075                1080                1085
Thr Val Arg Lys Lys Glu Glu Tyr Lys Met Ala Leu Tyr Asn Leu Tyr
    1090                1095                1100
Pro Gly Val Phe Glu Thr Val Glu Met Leu Pro Ser Lys Ala Gly Ile
1105                1110                1115                1120
Trp Arg Val Glu Cys Leu Ile Gly Glu His Leu His Ala Gly Met Ser
                1125                1130                1135
Thr Leu Phe Leu Val Tyr Ser Asn Lys Cys Gln Thr Pro Leu Gly Met
            1140                1145                1150
Ala Ser Gly His Ile Arg Asp Phe Gln Ile Thr Ala Ser Gly Gln Tyr
        1155                1160                1165
Gly Gln Trp Ala Pro Lys Leu Ala Arg Leu His Tyr Ser Gly Ser Ile
    1170                1175                1180
Asn Ala Trp Ser Thr Lys Glu Pro Phe Ser Trp Ile Lys Val Asp Leu
1185                1190                1195                1200
Leu Ala Pro Met Ile Ile His Gly Ile Lys Thr Gln Gly Ala Arg Gln
                1205                1210                1215
Lys Phe Ser Ser Leu Tyr Ile Ser Gln Phe Ile Ile Met Tyr Ser Leu
            1220                1225                1230
Asp Gly Lys Lys Trp Gln Thr Tyr Arg Gly Asn Ser Thr Gly Thr Leu
        1235                1240                1245
Met Val Phe Phe Gly Asn Val Asp Ser Ser Gly Ile Lys His Asn Ile
    1250                1255                1260
Phe Asn Pro Pro Ile Ile Ala Arg Tyr Ile Arg Leu His Pro Thr His
1265                1270                1275                1280
Tyr Ser Ile Arg Ser Thr Leu Arg Met Glu Leu Met Gly Cys Asp Leu

1285                    1290                    1295
Asn Ser Cys Ser Met Pro Leu Gly Met Glu Ser Lys Ala Ile Ser Asp
            1300                    1305                    1310
Ala Gln Ile Thr Ala Ser Ser Tyr Phe Thr Asn Met Phe Ala Thr Trp
        1315                    1320                    1325
Ser Pro Ser Lys Ala Arg Leu His Leu Gln Gly Arg Ser Asn Ala Trp
    1330                    1335                    1340
Arg Pro Gln Val Asn Asn Pro Lys Glu Trp Leu Gln Val Asp Phe Gln
1345                    1350                    1355                    1360
Lys Thr Met Lys Val Thr Gly Val Thr Thr Gln Gly Val Lys Ser Leu
                1365                    1370                    1375
Leu Thr Ser Met Tyr Val Lys Glu Phe Leu Ile Ser Ser Ser Gln Asp
            1380                    1385                    1390
Gly His Gln Trp Thr Leu Phe Phe Gln Asn Gly Lys Val Lys Val Phe
        1395                    1400                    1405
Gln Gly Asn Gln Asp Ser Phe Thr Pro Val Val Asn Ser Leu Asp Pro
    1410                    1415                    1420
Pro Leu Leu Thr Arg Tyr Leu Arg Ile His Pro Gln Ser Trp Val His
1425                    1430                    1435                    1440
Gln Ile Ala Leu Arg Met Glu Val Leu Gly Cys Glu Ala Gln Asp Leu
                1445                    1450                    1455
Tyr

<210> 29
<211> 1533
<212> PRT
<213> Artificial Sequence

<220>
<221> SIGNAL
<222> 1..19

<220>
<223> 29 AC-6rs aa incl. signal sequence

<400> 29
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr

195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asp Pro Leu Ala Trp
        755                 760                 765
Asp Asn His Tyr Gly Thr Gln Ile Pro Lys Glu Glu Trp Lys Ser Gln
    770                 775                 780
Glu Lys Ser Pro Glu Lys Thr Ala Phe Lys Lys Lys Asp Thr Ile Leu
785                 790                 795                 800
Ser Leu Asn Ala Cys Glu Ser Asn His Ala Ile Ala Ala Ile Asn Glu
                805                 810                 815
Gly Gln Asn Lys Pro Glu Ile Glu Val Thr Trp Ala Lys Gln Gly Arg
            820                 825                 830
Thr Glu Arg Leu Cys Ser Gln Asn Pro Pro Val Leu Lys Arg His Gln
        835                 840                 845
Arg Glu Ile Thr Arg Thr Thr Leu Gln Ser Asp Gln Glu Glu Ile Asp
    850                 855                 860
Tyr Asp Asp Thr Ile Ser Val Glu Met Lys Lys Glu Asp Phe Asp Ile
865                 870                 875                 880
Tyr Asp Glu Asp Glu Asn Gln Ser Pro Arg Ser Phe Gln Lys Lys Thr
                885                 890                 895
Arg His Tyr Phe Ile Ala Ala Val Glu Arg Leu Trp Asp Tyr Gly Met
            900                 905                 910
Ser Ser Ser Pro His Val Leu Arg Asn Arg Ala Gln Ser Gly Ser Val
        915                 920                 925
Pro Gln Phe Lys Lys Val Val Phe Gln Glu Phe Thr Asp Gly Ser Phe
    930                 935                 940
Thr Gln Pro Leu Tyr Arg Gly Glu Leu Asn Glu His Leu Gly Leu Leu
945                 950                 955                 960
Gly Pro Tyr Ile Arg Ala Glu Val Glu Asp Asn Ile Met Val Thr Phe
                965                 970                 975
Arg Asn Gln Ala Ser Arg Pro Tyr Ser Phe Tyr Ser Ser Leu Ile Ser
            980                 985                 990
Tyr Glu Glu Asp Gln Arg Gln Gly Ala Glu Pro Arg Lys Asn Phe Val
        995                 1000                1005
Lys Pro Asn Glu Thr Lys Thr Tyr Phe Trp Lys Val Gln His His Met
    1010                1015                1020
Ala Pro Thr Lys Asp Glu Phe Asp Cys Lys Ala Trp Ala Tyr Phe Ser
1025                1030                1035                1040
Asp Val Asp Leu Glu Lys Asp Val His Ser Gly Leu Ile Gly Pro Leu
                1045                1050                1055
Leu Val Cys His Thr Asn Thr Leu Asn Pro Ala His Gly Arg Gln Val
            1060                1065                1070
Thr Val Gln Glu Phe Ala Leu Phe Phe Thr Ile Phe Asp Glu Thr Lys
        1075                1080                1085
Ser Trp Tyr Phe Thr Glu Asn Met Glu Arg Asn Cys Arg Ala Pro Cys
    1090                1095                1100
Asn Ile Gln Met Glu Asp Pro Thr Phe Lys Glu Asn Tyr Arg Phe His
1105                1110                1115                1120
Ala Ile Asn Gly Tyr Ile Met Asp Thr Leu Pro Gly Leu Val Met Ala
                1125                1130                1135
Gln Asp Gln Arg Ile Arg Trp Tyr Leu Leu Ser Met Gly Ser Asn Glu
            1140                1145                1150
Asn Ile His Ser Ile His Phe Ser Gly His Val Phe Thr Val Arg Lys
        1155                1160                1165
Lys Glu Glu Tyr Lys Met Ala Leu Tyr Asn Leu Tyr Pro Gly Val Phe
    1170                1175                1180
Glu Thr Val Glu Met Leu Pro Ser Lys Ala Gly Ile Trp Arg Val Glu
1185                1190                1195                1200
Cys Leu Ile Gly Glu His Leu His Ala Gly Met Ser Thr Leu Phe Leu

1205                1210                1215
Val Tyr Ser Asn Lys Cys Gln Thr Pro Leu Gly Met Ala Ser Gly His
        1220                1225                1230
Ile Arg Asp Phe Gln Ile Thr Ala Ser Gly Gln Tyr Gly Gln Trp Ala
    1235                1240                1245
Pro Lys Leu Ala Arg Leu His Tyr Ser Gly Ser Ile Asn Ala Trp Ser
    1250                1255                1260
Thr Lys Glu Pro Phe Ser Trp Ile Lys Val Asp Leu Leu Ala Pro Met
1265                1270                1275                1280
Ile Ile His Gly Ile Lys Thr Gln Gly Ala Arg Gln Lys Phe Ser Ser
                1285                1290                1295
Leu Tyr Ile Ser Gln Phe Ile Ile Met Tyr Ser Leu Asp Gly Lys Lys
        1300                1305                1310
Trp Gln Thr Tyr Arg Gly Asn Ser Thr Gly Thr Leu Met Val Phe Phe
        1315                1320                1325
Gly Asn Val Asp Ser Ser Gly Ile Lys His Asn Ile Phe Asn Pro Pro
    1330                1335                1340
Ile Ile Ala Arg Tyr Ile Arg Leu His Pro Thr His Tyr Ser Ile Arg
1345                1350                1355                1360
Ser Thr Leu Arg Met Glu Leu Met Gly Cys Asp Leu Asn Ser Cys Ser
                1365                1370                1375
Met Pro Leu Gly Met Glu Ser Lys Ala Ile Ser Asp Ala Gln Ile Thr
        1380                1385                1390
Ala Ser Ser Tyr Phe Thr Asn Met Phe Ala Thr Trp Ser Pro Ser Lys
        1395                1400                1405
Ala Arg Leu His Leu Gln Gly Arg Ser Asn Ala Trp Arg Pro Gln Val
    1410                1415                1420
Asn Asn Pro Lys Glu Trp Leu Gln Val Asp Phe Gln Lys Thr Met Lys
1425                1430                1435                1440
Val Thr Gly Val Thr Thr Gln Gly Val Lys Ser Leu Leu Thr Ser Met
                1445                1450                1455
Tyr Val Lys Glu Phe Leu Ile Ser Ser Ser Gln Asp Gly His Gln Trp
        1460                1465                1470
Thr Leu Phe Phe Gln Asn Gly Lys Val Lys Val Phe Gln Gly Asn Gln
        1475                1480                1485
Asp Ser Phe Thr Pro Val Val Asn Ser Leu Asp Pro Pro Leu Leu Thr
    1490                1495                1500
Arg Tyr Leu Arg Ile His Pro Gln Ser Trp Val His Gln Ile Ala Leu
1505                1510                1515                1520
Arg Met Glu Val Leu Gly Cys Glu Ala Gln Asp Leu Tyr
                1525                1530

<210> 30
<211> 4371
<212> DNA
<213> Artificial Sequence

<220>
<223> AC-6rs-REF na incl. signal sequence without stop codon

<220>
<221> sig_peptide
<222> 1..57

<400> 30
atgcagatcg agctgtctac ctgcttcttc ctgtgcctgc tgcggttctg cttcagcgcc      60

acccggcggt actacctggg cgccgtggaa ctgagctggg actacatgca gagcgacctg     120

ggcgagctgc ccgtggacgc cagattcccc ccaagagtgc ccaagagctt ccccttcaac     180

acctccgtgg tgtacaagaa aaccctgttc gtcgagttca ccgaccacct gttcaatatc     240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gcctcggagc 2280

ttctcccaga acccccccgt gctgaagcgg caccagagag agatcacccg gaccaccctg 2340

cagagcgacc aggaagagat cgactacgac gacaccatca gcgtcgagat gaagaaagag 2400

gacttcgaca tctacgacga ggacgagaac cagagccccc ggtccttcca gaagaaaacc 2460

cggcactact ttatcgccgc cgtggaacgg ctgtgggact acggcatgag cagcagcccc 2520

cacgtgctgc ggaatagagc ccagagcggc agcgtgcccc agttcaagaa agtggtgttc 2580

caggagttca ccgacggcag cttcacccag cctctgtacc ggggcgagct gaacgagcac 2640

ctgggcctgc tgggccccta catccgggcc gaggtggaag ataacatcat ggtcaccttc 2700

cggaaccagg ccagccggcc ctacagcttc tacagcagcc tgatctccta cgaagaggac 2760

cagcggcagg gcgccgagcc ccggaagaac ttcgtgaagc ccaacgagac aaagacctac 2820

ttctggaagg tgcagcacca catggccccc accaaggacg aattcgactg caaggcctgg 2880

gcctacttca gcgacgtgga cctggaaaag gacgtgcaca gcggcctgat cggccccctg 2940

ctcgtgtgcc acaccaacac cctgaacccc gcccacggcc ggcaggtcac agtgcaggaa 3000

tttgccctgt tcttcaccat cttcgacgag actaagagct ggtacttcac cgagaacatg 3060

gaacggaact gcagagcccc ctgcaacatc cagatggaag atcccacctt caaagagaac 3120

taccggttcc acgccatcaa tggctacatc atggacaccc tgcccggcct ggtcatggcc 3180

caggaccaga gaatccggtg gtatctgctg agcatgggca gcaacgagaa catccacagc 3240

atccacttca gcggccacgt gttcaccgtg cggaagaaag aagagtacaa gatggccctg 3300

tacaacctgt accccggcgt gttcgagaca gtggaaatgc tgcccagcaa ggccggcatc 3360

tggcgggtgg aatgtctgat cggcgagcat ctgcacgccg gcatgtccac cctgtttctg 3420

gtgtacagca acaagtgcca gacccccctg ggcatggcca gcggccacat ccgggatttc 3480

cagatcaccg cctccggcca gtacggccag tgggccccta aactggcccg gctgcactac 3540

agcggcagca tcaacgcctg gtccaccaaa gagcccttca gctggatcaa ggtggacctg 3600

ctggccccca tgattatcca cggcatcaag acacagggcg ccagacagaa gttcagcagc 3660

ctgtacatca gccagttcat catcatgtac agcctggatg gcaagaagtg gcagacctac 3720

cggggcaaca gcaccggcac cctgatggtg ttcttcggca acgtggacag cagcggcatc 3780

aagcacaaca tcttcaaccc ccccatcatt gcccggtaca tccggctgca ccccacccac 3840

tacagcatcc ggtccaccct gcggatggaa ctgatgggct gcgacctgaa cagctgctcc 3900

atgcctctgg gcatggaaag caaggccatc agcgacgccc agatcacagc cagcagctac 3960

ttcaccaaca tgttcgccac ctggtcccca tccaaggcca gactgcatct gcagggcaga 4020

agcaatgcct ggcggcctca ggtcaacaac cccaaagaat ggctccaggt ggacttccag     4080

aaaaccatga aggtcacagg cgtgaccacc cagggcgtga agtccctgct gacctctatg     4140

tacgtgaaag agttcctgat ctccagcagc caggacggcc accagtggac cctgttcttt     4200

cagaacggca aagtgaaagt gttccagggc aaccaggact ccttcacccc cgtggtcaac     4260

tccctggacc ccccactgct gaccagatac ctgagaatcc acccccagag ctgggtgcac     4320

cagatcgccc tgagaatgga agtgctggga tgcgaggccc aggatctgta c              4371

<210> 31
<211> 9
<212> PRT
<213> Artificial Sequence

<220>
<223> merging sequence in V1

<400> 31
Ser Val Glu Met Lys Lys Glu Asp Phe
1               5

<210> 32
<211> 20
<212> PRT
<213> Homo sapiens

<220>
<223> sequence N-terminal to processing sequence and including 2 aa of
      processing sequence, i.e., sequence N-terminal of thrombin
      cleavage site

<400> 32
Asp Ser Tyr Glu Asp Ile Ser Ala Tyr Leu Leu Ser Lys Asn Asn Ala
1               5                   10                  15
Ile Glu Pro Arg
            20

<210> 33
<211> 5
<212> PRT
<213> Homo sapiens

<220>
<223> sequence from the B domain

<400> 33
Asp Pro Leu Ala Trp
1               5

<210> 34
<211> 4
<212> PRT
<213> Artificial Sequence

<220>
<223> thrombin cleavage site in V1

<400> 34
Pro Arg Ser Val
1

<210> 35
<211> 6
<212> PRT
<213> Artificial Sequence


<220>
<223> sequence in V2

<400> 35
Pro Arg Val Ala Ser Val
1               5

<210> 36
<211> 4
<212> PRT
<213> Artificial Sequence


<220>
<223> sequence in V3

<400> 36
Ser Cys Ser Val
1

<210> 37
<211> 14
<212> PRT
<213> Artificial Sequence


<220>
<223> sequence in V4

<400> 37
Ser Cys Asp Pro Arg Ile Arg Ser Val Val Ala Gln Ser Val
1               5                   10

<210> 38
<211> 10
<212> PRT
<213> Artificial Sequence


<220>
<223> insertion in V4

<400> 38
Asp Pro Arg Ile Arg Ser Val Val Ala Gln
1               5                   10